# EUROPEAN PATENT APPLICATION

(11) **EP 2 292 684 A2**
(43) Date of publication of application: **09.03.2011**
(21) Application number: 10196921.0
(22) Date of filing: 20.12.2006
(51) Int. Cl.: C08J 11/24, C07C 67/03, C07C 69/82, B01J 19/18, C07C 69/80, C07C 51/23, C07C 63/15, C07C 63/16, C07C 63/24, C07C 63/26, C07C 63/38

(54) **Ethanolysis of pet to form det and oxidation thereof**

(30) Priority: 29.12.2005 US 754772 P; 29.12.2005 US 754949 P; 29.12.2005 US 754698 P
(62) Divisional of application: 06846707.5
(71) Applicant: BP Corporation North America Inc., Warrenville, Illinois 60555 (US)
(72) Inventor: Anderson, Ronald L., St. Charles, IL 60175 (US); Sikkenga, David L., Wheaton, IL 60187 (US)
(74) Representative: Hamer, Christopher K.

(57) **Abstract**

A process for ethanolysis of PET is disclosed wherein a feed comprising PET is reacted with ethanol and recovering ethylene glycol and an aromatic diethyl ester such as diethyl isophthalate and/or diethyl terephthalate. PET, or a terpolymer comprising terephthalate monomer and ethylene glycol monomers, is reacted with ethanol and ethanol, diethyl terephthalate, ethylene glycol and optionally diethyl isophthalate are recovered. Recovered diethyl components can be subjected to liquid-phase oxidation to produce aromatic carboxylic acid. Acetic acid may also produced via liquid-phase oxidation of recovered diethyl components. The aromatic carboxylic acid can be used to form polymer.

## Description

### Background of the Invention

This invention provides a process for oxidation of aromatic ethyl esters and for recycling poly(ethylene terephthalate) ("PET") and other polymers comprising ethylene monomers and ester monomers, particularly aromatic ester monomers. The invention also provides a process for recycling waste polymer having PET and, optionally, other polymers. The invention provides a process for recovering ethylene glycol and ethyl esters from such waste polymers and producing polymers therefrom. The invention also provides a feedstock with aromatic ethyl ester component useful for the production of aromatic carboxylic acids and a method for producing acetic acid and aromatic carboxylic acids.

PET and other copolymers, for example poly(ethylene isophthalate) ("PEI"), poly(ethylene naphthalate) ("PEN") and others, are commonly used in films, fibers, packaging and numerous other applications. The wide use of such polymers has led to increased interest in recycling products made from such polymers. Many jurisdictions require or offer incentives for recycling polymers. Also consumers and consumer oriented businesses are increasingly interested in using or selling recyclable products. As used herein, "polymers" includes copolymers. As used herein, "ester-ethylene polymer" means a polymer having at least ester monomers and ethylene monomers and which may include other monomer components. As used herein, "aromatic ester-ethylene polymer" refers to an ester-ethylene polymer wherein the ester monomers include ester monomers having one or more aromatic rings.

One method of recycling such polymer products is by blending waste polymer with virgin polymer. Unfortunately, the polymer products, and consequently the waste polymer, often contain significant amounts of impurities which greatly limits the utility if such a blending process. Often waste polymer includes adhesives, metals, dyes and many other contaminants that make such waste unsuitable for many recycle processes. In some cases, polymer products contain multiple polymers or copolymers which increase difficulty for recycling. For example, in the case of PET, often PEI and phthalic anhydride derivatives are considered impurities detrimental to recycling. For products which include several different types of polymers, waste/virgin polymer blending can be inappropriate. Furthermore, the blend of waste and virgin product often results in significant degradation by the waste product making the resulting blended polymer unsuitable for many applications.

For recycling PET, an alternative recycling method is methanolysis wherein the PET is reacted with methanol to produce dimethyl terephthalate ("DMT") and ethylene glycol. Although such methanolysis processes can tolerate slightly greater amounts of impurities, such processes are still extremely limited in their ability to recycle impure products. Additionally, products containing several different types of polymers can be entirely unsuitable or significantly diminish the efficacy of methanolysis processes, for example, products containing a mix of PET and polyvinylchloride or other halogenated polymers or polymers containing significant amount of metals. Methanolysis of PET has other significant disadvantages including a difficult separation process to extract DMT from ethylene glycol. Additionally, storage and handling of DMT can be difficult due to its high melting point.

Ethanolysis is the transesterification of PET with ethanol to produce ethylene glycol and diethyl terephthalate (DET). In some disclosures of the methanolysis of PET, reference has been made to the possibility of using other lower alcohols, however, there is no disclosure of how such a process could be conducted using ethanol. Additionally, there is no appreciation of the significant differences between methanolysis of PET and ethanolysis of PET. Nor is there any appreciation of the significant advantages that ethanolysis of PET can provide over methanolysis. For example, DET can be oxidized to produce terephthalic acid ("TA") via liquid-phase oxidation in existing operations for producing TA via liquid-phase oxidation of paraxylene. For further example, DET product has a lower melting point than DMT so that liquid phase operations, such as liquid-liquid separation from ethylene glycol can be performed more readily. The lower melting point of DET product can also make storage and handling easier compared to DMT.

Another method of recycling PET is depolymerization. In depolymerization, the ester bond is broken and the polymer is reduced to its monomer components. Typically it is desirable to purify the monomers. However, in existing depolymerization methods, such purification can make the recycled polymer more difficult to make and more expensive than virgin polymer.

Reaction of PET with ethylene glycol to form bis(hydroxyethyl) terephthalate (BHET) is one way to recycle PET by depolymerization. Purification methods for the resulting BHET monomer are limited however, since it has low volatility and polymerizes to PET at elevated temperatures. These properties make distillation of the BHET monomer impractical, which means that a fairly clean recycled PET feed stream must be used for depolymerization by glycolysis. This severely limits the utility of glycolysis as a PET recycle process.

We have discovered a process for recycling waste polymer, particularly PET and other ester-ethylene polymers by ethanolysis to form ethyl esters and ethylene glycol, oxidizing the resulting ethyl ester to form carboxylic acid and acetic acid from which PET and other polymers can be created.

Aromatic carboxylic acids such as benzoic, phthalic, terephthalic, isophthalic, trimellitic, pyromellitic, trimesic and naphthalene dicarboxylic acids are important intermediates for many chemical and polymer products. Terephthalic and isophthalic acids are used to make PET and PEI, respectively. Naphthalene dicarboxylic acid is used to make PEN. Phthalic acid is widely used, in its anhydride form, to make plasticizers, dyes, perfumes, saccharin and many other chemical compounds.

Aromatic carboxylic acids can commonly be made by oxidizing the corresponding dimethyl aromatic hydrocarbon precursor. For example, terephthalic acid is typically made by oxidizing paraxylene and isophthalic acid is typically made by oxidizing metaxylene. Phthalic acid can be made by oxidizing orthoxylene. Naphthalene dicarboxylic acid is typically made by oxidizing 2,6-dimethylnaphthalene.

An example of such processes can be found in U.S. Patent No. 2,833,816, hereby incorporated by reference, which discloses the liquid phase oxidation of xylene isomers into corresponding benzene dicarboxylic acids in the presence of bromine using a catalyst having cobalt and manganese components. As further example, U.S. Patent No. 5,103,933, incorporated by reference herein, discloses that liquid phase oxidation of dimethyl naphthalenes to naphthalene dicarboxylic acids can also be accomplished in the presence of bromine and a catalyst having cobalt and manganese components.

Typically, aromatic carboxylic acids are purified in a subsequent process. For example, a process involving contacting crude aromatic carboxylic acid with a catalyst and hydrogen in a reducing environment as described, for example, in U.S. Patent No. 3,584,039, U.S. Patent No. 4,892,972, and U.S. Patent No. 5,362,908.

Subsequent purification processes typically include contacting a solution of the crude aromatic carboxylic acid product of the oxidation with hydrogen and a catalyst under reducing conditions. The catalyst used for such purification typically comprises one or more active hydrogenation metals such as ruthenium, rhodium, palladium, or platinum, on a suitable support, for example, carbon or titania.

As used herein, "aromatic hydrocarbon" means a molecule composed of carbon atoms and hydrogen atoms, and having one or more aromatic ring, for example a benzene or naphthalene ring. For purposes of this application, "aromatic hydrocarbon" includes such molecules having one or more hetero atoms such as oxygen or nitrogen atoms. "Methyl aromatic hydrocarbon" means an aromatic hydrocarbon molecule having one or more methyl groups attached to one or more aromatic rings. "Aromatic ethyl esters" means the ethyl esters of aromatic acids having one or more ethyl groups. As used herein, "aromatic carboxylic acid" means an aromatic acid having one or more carboxylic acid groups.

Liquid phase oxidation of dimethyl aromatic hydrocarbons to aromatic carboxylic acid is commonly conducted using a reaction mixture comprising methyl aromatic hydrocarbons and a solvent in the presence of a source of molecular oxygen. Typically, the solvent comprises a C₁-C₈ monocarboxylic acid, for example acetic acid or benzoic acid, or mixtures thereof with water. Such processes generally involve the addition of a certain amount of make-up solvent because some solvent is lost for example due to burning, side reactions, separation inefficiencies or other process losses. Such solvent loss can be considerably undesirable and, often, significant efforts are made to minimize losses and maximize solvent recovery so as to reduce the amount of make-up solvent required.

A catalyst is also present in the oxidation reaction mixture. Typically, the catalyst comprises a promoter, for example bromine, and at least one suitable heavy metal component. Suitable heavy metals include heavy metals with atomic weight in the range of about 23 to about 178. Examples include cobalt, manganese, vanadium, molybdenum,' chromium, iron, nickel, zirconium, hafnium or a lanthanoid metal such as cerium. Suitable forms of these metals include for example, acetates, hydroxides, and carbonates.

A source of molecular oxygen is also introduced into the reaction mixture. Typically, oxygen gas is used as a source of molecular oxygen and is bubbled or otherwise mixed into the liquid phase reaction mixture. Air is generally used to supply the oxygen. Generally, a minimum of 1.5 mols of O₂ is needed for each methyl group to convert a methyl aromatic hydrocarbon to the corresponding aromatic carboxylic acid with the co-production of one mols of H₂O. For example, to covert one mol dimethyl aromatic hydrocarbon to one mol aromatic dicarboxylic acid, a minimum of 3.0 mols of O₂ is needed and two mols H₂O is produced.

We have discovered that aromatic ethyl esters can be suitable feedstock for the production of aromatic carboxylic acids and may even be used in the same or similar processes employed for producing aromatic carboxylic acids from methyl aromatic hydrocarbons. The use of aromatic ethyl esters is particularly useful when the reaction solvent includes acetic acid because, in the oxidation process, aromatic ethyl esters oxidize to form the corresponding aromatic dicarboxylic acid and acetic acid. In cases where the solvent includes acetic acid, aromatic ethyl esters can be used to reduce or even eliminate the need for make-up solvent.

If methanolysis of PET is employed to produce DMT and ethylene glycol, the resulting DMT would typically be converted to TA and methanol via hydrolysis. Unfortunately, such hydrolysis requires special equipment both for the process and for recovery of the methanol byproduct. TA is more commonly produced by the liquid-phase oxidation of paraxylene but DMT is unsuitable for use in such liquid-phase oxidation processes because, among other reasons, the methyl groups are converted to CO, CO₂ methyl acetate or other undesirable co-products.. In contrast, DET is suitable for liquid-phase oxidation processes which are also capable of converting paraxylene to TA.

### Summary of the Invention

We have discovered that aromatic ethyl esters are useful as feedstock for production of aromatic carboxylic acids. Aromatic ethyl esters, preferably including aromatic diethyl esters, can be used in liquid phase oxidation processes to produce aromatic carboxylic acids. Such a mechanism is particularly useful in the case of DET, diethyl isophthalate ("DEI") and diethyl naphthalate ("DEN") which can be used in existing xylene oxidation processes to produce terephthalic acid and isophthalic acids, respectively. Aromatic ethyl esters can also be used to produce acetic acid or even co-produce aromatic carboxylic acid and acetic acid. Aromatic ethyl esters can be recovered by recycling polymer products derived from aromatic carboxylic acids and the carboxylic acids can be used to form polymers as disclosed in our parent applications entitled "Ethanolysis of PET and Production of Diethyl Terephthalate" and "PET Recycle Process" both filed on December 29, 2005, incorporated by reference herein. In particular, ethanolysis can be used to recover DET and DEI from PET and PEI respectively.

In some embodiments, this invention provides a feedstock for the production of aromatic carboxylic acid comprising at least one aromatic ethyl ester, preferably aromatic diethyl ester. Measured on the basis of total aromatic carboxylic acid precursors for the desired aromatic carboxylic acid or acids, the feedstock preferably comprises at least about 1 wt% of the at least one aromatic ethyl ester, more preferably at least about 5 wt% and more preferably at least about 10 wt% of the at least one aromatic ethyl ester. The aromatic diethyl ester is preferably DET, DEI, DEN or a combination thereof. The feedstock can also comprise a dimethyl aromatic hydrocarbon for example, paraxylene.

In another embodiment, this invention provides a method of producing terephthalic acid comprising oxidizing diethyl terephthalate to form terephthalic acid.

In other embodiments, this invention provides a method of producing aromatic carboxylic acids comprising the step of reacting in a reaction zone at least one aromatic ethyl ester, preferably aromatic diethyl ester, and oxygen in the presence of a solvent comprising acetic acid. Measured on the basis of total aromatic carboxylic acid precursors present in the reaction zone for the desired aromatic carboxylic acid or acids, the at least one aromatic ethyl ester is preferably present at at least about 1 wt%, more preferably at least about 5 wt%, more preferably at least about 10 wt%. The aromatic diethyl ester is preferably DET, DEI, DEN or a combination thereof. The method can further comprise the step of reacting in the reaction zone at least one dimethyl aromatic hydrocarbon and oxygen in the presence of the solvent. The at least one dimethyl aromatic hydrocarbon is preferably paraxylene. Preferably a catalyst comprising at least one heavy metal is present in the reaction zone. The at least one heavy metal preferably includes at least one of cobalt or manganese. The catalyst preferably also comprises a halogen compound, preferably bromine.

In some other embodiments, this invention provides a method for producing acetic acid comprising the step of reacting in a reaction zone at least one aromatic ethyl ester, preferably aromatic diethyl ester, in the presence of oxygen and, optionally, water. Preferably, a catalyst comprising at least one heavy metal is present in the reaction zone. The at least one heavy metal preferably includes at least one of cobalt or manganese. The catalyst preferably also comprises a halogen compound, preferably bromine. Preferably, the at least one aromatic diethyl ester includes DET, DEI, DEN or a combination thereof.

In other embodiments, this invention provides a method of co-producing aromatic carboxylic acid and acetic acid comprising reacting in a reaction zone a feedstock comprising a aromatic ethyl ester, preferably aromatic diethyl ester, with oxygen. The aromatic diethyl ester is preferably DET, DEI, DEN or a combination thereof. Optionally, at least one dimethyl aromatic hydrocarbon, preferably paraxylene, can be present in the reaction zone. Preferably a catalyst comprising at least one heavy metal is present in the reaction zone. The at least one heavy metal preferably includes at least one of cobalt or manganese. The catalyst preferably also comprises a halogen compound, preferably bromine.

We have discovered that recycling PET via ethanolysis can provide significant advantages over other recycling methods. Significantly, the product of ethanolysis of PET is DET and ethylene glycol. The separation of DET and ethylene glycol from the reaction products and from each other is significantly different and more desirable than the separation of DMT and ethylene glycol. Furthermore, DET can be used in many existing plants which produce TA via liquid-phase oxidation of paraxylene. Additionally, because DET has a significantly lower melting point than DMT, DET can be handled, shipped and/or stored easily as a melt rather than as a solid. If operating in a liquid phase, generally, for a given temperature, use of ethanol as opposed to methanol permits operation at a lower pressure to achieve a desired concentration of alcohol in liquid phase. Operation at lower pressures can result in significant energy savings.

We have discovered that certain types of PET contain impurities that catalyze ethanolysis of PET. Additionally, titanium, preferably in the form of an organic titanate, is an effective catalyst. We have also found that ethanolysis of PET can be conducted so as to be tolerant of the presence of some water which allows the use of fuel grade ethanol.

Also, we have discovered that, unlike some methanolysis recycling processes which can require quenching of catalyst after the reaction to avoid undesirable back-reactions including reaction of DMT with ethylene glycol., ethanolysis catalysts can be kept active without detrimental effect upon product recovery. This allows the option of reusing the catalyst without reactivation steps.

In one embodiment, this invention provides a process for recycling poly(ethylene terephthalate). The process comprises the steps of combining in a reaction zone poly(ethylene terephthalate) with ethanol to form a reaction mixture; reacting the reaction mixture at a temperature in the range from about 180°C to about 300°C to form a reaction product mixture; recovering from the reaction product mixture a first fraction comprising recovered ethanol; recovering from the reaction product mixture a second fraction comprising ethylene glycol; and recovering from the reaction product mixture a third fraction comprising diethyl terephthalate.

Preferably, the step of recovering from the reaction product mixture a first fraction comprising recovered ethanol is performed in a first separation zone and the steps of recovering from the reaction product mixture a second fraction comprising ethylene glycol and recovering from the reaction product mixture a third fraction comprising diethyl terephthalate are performed in a second separation zone.

Some embodiments also include the steps of separating the second fraction into a first stream comprising a major portion of diethyl terephthalate and a second stream comprising ethylene glycol; returning at least a portion of the first stream to the second separation zone; and recovering ethylene glycol from the second stream in a third separation zone. Preferably the step of separating the second fraction is performed using liquid-liquid separation. Optionally, the step of separating the second fraction can comprise the step of adding water to at least a portion of the second fraction. In some embodiments, the first separation zone comprises a first distillation column and the second separation zone comprises a second distillation column. Preferably, the first distillation column is operated at about atmospheric pressure and the second distillation column is operated at a pressure less than atmospheric pressure. Optionally, at least a portion of the recovered ethanol in the first fraction can be directed to the reaction zone.

In some embodiments, catalyst is supplied to the reaction zone and, preferably, the catalyst is selected from the group consisting of catalyzing impurities present in PET, copper phthalocyanine, zinc acetate, cobalt acetate, manganese acetate, magnesium acetate, titanium(IV) isopropoxide or other organic titanates, and combinations thereof. Optionally, water can be supplied to the reaction zone for example, by use of fuel grade ethanol. Preferably, in such embodiments, the catalyst comprises titanium, preferably in the form of organic titanates.

Some embodiments include the step of recovering from the reaction product mixture a fourth fraction comprising catalyst and PET oligomers. Preferably at least a portion of the fourth fraction is directed to the reaction zone.

Another embodiment of the invention provides an apparatus for the recycle of poly(ethylene terephthalate). The apparatus comprises a reactor capable of reacting poly(ethylene terephthalate) and ethanol and forming a reaction product mixture; a flash drum or an atmospheric distillation column adapted to recover ethanol from the reaction product mixture; and a vacuum distillation column adapted to recover diethyl terephthalate from the reaction product mixture. Optionally, the apparatus can include a decanting vessel adapted to receive a portion of the reaction product mixture.

Some embodiments provide a process for the production of diethyl terephthalate. Such process comprises the steps of reacting poly(ethylene terephthalate) and ethanol in a reaction zone to form a reaction product mixture comprising ethanol, poly(ethylene terephthalate), diethyl terephthalate and ethylene glycol; separating from the reaction product mixture a first fraction comprising ethanol, a second fraction comprising a diethyl terephthalate - ethylene glycol azeotrope and a third fraction comprising diethyl terephthalate; recovering from the azeotrope a stream comprising a major portion of diethyl terephthalate; and directing at least a portion of the stream to the separation step. Preferably, a catalyst is present in the reaction zone. The catalyst is more preferably selected from the group consisting of catalyzing impurities present in the PET, copper phthalocyanine, zinc acetate, cobalt acetate, manganese acetate, magnesium acetate, titanium(IV) isopropoxide or other organic titanates and combinations thereof. In some embodiments, the invention provides a process for producing diethyl terephthalate and diethyl isophthalate. Such process comprises the steps of reacting in a reaction zone ethanol with a feed comprising poly(ethylene terephthalate) and poly(ethylene isophthalate) to form a reaction product mixture; recovering from the reaction product mixture a first fraction comprising ethanol; recovering from the reaction product mixture a second fraction comprising ethylene glycol; and recovering from the reaction product mixture a third fraction comprising diethyl terephthalate and diethyl isophthalate. Preferably, a catalyst is present in the reaction zone. The catalyst is more preferably selected from the group consisting of catalyzing impurities present in the PET, copper phthalocyanine, zinc acetate, titanium(IV) isopropoxide or other organic titanates or combinations thereof. Optionally, water may be present in the reaction zone. Preferably, organic titanates are present in the reaction zone. Preferably, the ethanol in the reaction zone comprises fuel grade ethanol.

We have discovered that a feed including PET can be reacted with ethanol to form diethyl esters which can be oxidized to form aromatic carboxylic acid which can then be used to form polymers. In particular, PET can be reacted with ethanol to form ethylene glycol and diethyl terephthalate which can be fed to existing liquid phase oxidation processes for the production of terephthalic acid which can be used to form PET. The recycle process is tolerant of many contaminants allowing use of a broad range of waste PET. The recycle method allows the recycle of PET and other polymers without degradation of the final recycled polymer product.

In some embodiments the invention provides a process for recycling PET. The process comprises the steps of reacting, in a first reaction zone, a first feed comprising PET with ethanol to form a first reaction product mixture; recovering from the first reaction product mixture aromatic ethyl esters; oxidizing, in a second reaction zone, a second feed comprising at least a portion of the aromatic ethyl esters to form aromatic carboxylic acid; and reacting, in a third reaction zone, at least a portion of the aromatic carboxylic acid and ethylene glycol to form a polymer comprising PET. The first feed can comprise at least 1000 ppmw polyvinylchloride (on a PET basis). The second feed preferably includes dimethyl aromatic hydrocarbon precursors of the desired aromatic carboxylic acid. At least a portion of the first reaction product mixture can be contacted with an ion exchange resin to remove at least a portion of soluble contaminants present in the first reaction product mixture. The first reaction product mixture can be brought to a temperature of from about 50 C to about 120 C to simplify handling and processing.

The aromatic carboxylic acid can be purified before being used to form polymers. Ethanol used can be fuel grade ethanol.

In other embodiments, the invention provides a process for making PET from waste PET. The process comprises reacting in a first reaction zone a first feed comprising PET with ethanol to form a first product mixture; recovering DET from the first reaction product mixture; reacting in a second reaction zone at least a portion of the DET with oxygen in the presence of a solvent comprising low molecular weight monocarboxylic acid to form terephthalic acid; purifying at least a portion of the terephthalic acid in a hydrogenation reaction zone to form purified terephthalic acid; and producing PET using at least a portion of the purified terephthalic acid

### Brief Description of the Drawing

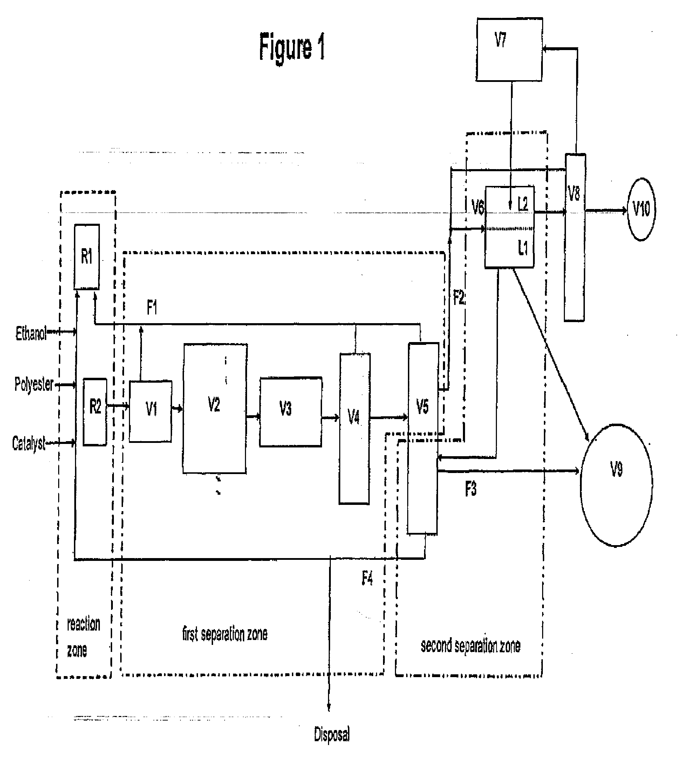

Figure 1 illustrates and embodiment of ethanolysis and product recovery in accordance with an embodiment of this invention.

### Description of the Preferred Embodiment(s)

This invention provides processes and apparatuses for the recycle of PET via ethanolysis and for the production of DET. Ethanolysis is the transesterification of PET with ethanol to produce ethylene glycol and DET. Various types and grades of PET can be recycled via ethanolysis including but not limited to brown flake, green flake, blue flake, clear flake, amber flake or mixtures thereof. The ability to use mixed PET flake is advantageous as such mixed flake is a more readily available feed than pure flake such as pure clear flake. In some embodiments, the PET to be recycled is in the form of PET bale which optionally can be ground and/or dissolved in a suitable solvent.

This invention also provides feedstocks useful for the production of aromatic carboxylic acids. Such feedstocks include one or more aromatic ethyl esters. Aromatic ethyl esters can be used alone as such feedstock. In a preferred embodiment, one or more aromatic ethyl esters are used as a component of a feedstock for the production of aromatic carboxylic acids. Aromatic ethyl esters are particularly useful as feedstock for liquid-phase oxidation processes to produce aromatic carboxylic acids.

This invention also provides a method for recycling PET and other polyesters by reacting waste polymer with ethanol to form ethylene glycol and ethyl esters which can be oxidized to corresponding carboxylic acids. The carboxylic acids, and optionally the ethylene glycol recovered from ethanolysis can be used to form the polyesters.

In some embodiments, the ethyl ester can be used as feed in existing oxidation processes for producing the corresponding carboxylic acid. For example, aromatic diethyl esters can be used in existing liquid phase oxidation processes for making aromatic dicarboxylic acids from aromatic dimethyl hydrocarbons. Once converted to aromatic dicarboxylic acid, it can be used in place of or together with aromatic dicarboxylic acids which did not originate from recycled polyester. This allows the use of recycled materials without any degradation of the final polyester product and without altering existing polymerization processes which create polyesters using aromatic carboxylic acids.

Ethanolysis is the transesterification of polyester with ethanol to produce ethyl esters and ethylene glycol. The ethyl esters can be converted to corresponding carboxylic acids which can be used to form the polymer using a polycondensation reaction process.

In particular embodiments the recycle process can use a wide range of polyester feed including many impure waste polyesters. In embodiments where the recycle is used to recycle waste PET a wide range of impure waste PET feeds can be used including but not limited to waste PET having other polyesters, having terpolymers, polyvinyl chloride, polyolefins, adhesives, heavy metals and many other impurities that can be unsuitable for other recycling processes.

Recycle of PET via ethanolysis produces DET and ethylene glycol. Ethanolysis is the transesterification of PET with ethanol to produce ethylene glycol and DET. Various types and grades of PET can be recycled via ethanolysis including but not limited to brown flake, green flake, blue flake, clear flake, amber flake or mixtures thereof. The ability to use mixed PET flake is advantageous as such mixed flake is a more readily available feed than pure flake such as pure clear flake. In some embodiments, the PET to be recycled is in the form of PET bale which optionally can be ground and/or dissolved in a suitable solvent.

Recycle of PET using ethanol can be conducted as a continuous or batch process to obtain DET and ethylene glycol or as a semi-batch process. An example of a semi-batch process would be batch ethanolysis of PET and continuous recovery process for recovering DET and ethylene glycol products from the batch reaction mixture. PET and ethanol are reacted in an ethanolysis reaction zone in the presence of a suitable catalyst. The resulting reaction product mixture is subjected to separation for product recovery. Such separation can be performed using numerous separation techniques known in the art. However, separation preferably includes distillation to recover ethanol, DET and ethylene glycol.

PET, typically in the form of consumer product waste or as waste flake, is preferably dissolved in a solvent. Any solvent which is not detrimental to the ethanolysis reaction can be used. However, it is preferable that the solvent include ethanol and/or distillation bottoms from the second separation zone. In one embodiment, the solvent includes a portion of the reaction product mixture obtained from the reaction zone. Optionally, dissolved PET feed may be filtered if needed to remove impurities, for example adhesives, which may be present in some feeds. The PET feed is reacted with ethanol in a reaction zone in the presence of a suitable catalyst. Ethanol can be combined with the PET feed in the reaction zone, upstream of the reaction zone, or using a combination thereof. Catalyst can be added in the reaction zone, combined with the PET feed, combined with ethanol, combined with solvent, may be present in the recycled bottoms stream, or combinations thereof.

PET feed may include other polymers and impurities, for example PEI, PEN, polyvinylchloride, polyolefins, heavy metals, dyes, plasticizers and many other compounds which are often used to form PET products or used in conjunction with PET. Generally, ethanolysis of PET, as described herein, is more tolerant of the presence of such other polymers and impurities than many other PET recycling processes. Advantageously, some other polymers are converted via ethanolysis to corresponding ethyl esters which may be converted to corresponding carboxylic acids which can be esterified and polymerized to form polymers. In some embodiments, at least a portion of other polymers present with PET are reacted with ethanol to form aromatic ethyl esters. Such aromatic ethyl esters can be oxidized to form aromatic carboxylic acids which can be esterified and polymerized to reform the polymers.

Ethanol used for ethanolysis can be industrial grade ethanol, however, we have discovered that fuel grade ethanol can be used effectively. Fuel grade ethanol typically contains more water than industrial grade ethanol and commonly contains a denaturant (typically a hydrocarbon or hydrocarbonaceous compound). In some embodiments of the invention paraxylene can be used as the denaturant. In such embodiments the paraxylene can be recovered from the reaction products and can be blended with the DET. Such embodiments are particularly advantageous for use in a liquid phase oxidation process for converting paraxylene to TA. Although the exact formulation of fuel grade ethanol varies, fuel grade ethanol can contain from about 0.25 to about 2.0 % by volume water but typically contains approximately 1 vol% water and from about 1 to 5 vol% denaturant. Fuel grade ethanol may also contain other compounds for example trace metallic compounds, gums and methanol. Although different jurisdictions may have different specifications for fuel grade ethanol, such variations are not expected to significantly impact ethanolysis of PET as described herein. ASTM D 4806 (Standard Specification for Denatured Fuel Ethanol for Blending with Gasoline for Use as Automotive Spark Ignition Engine Fuel) is an example of specifications for fuel grade ethanol commonly used in the United States.

We have found that ethanolysis can be effectively practiced despite the presence of the water, denaturant and other compounds in fuel grade ethanol. We have found that ethanolysis as taught herein can be practiced effectively using ethanol having up to about 5 wt% water. The ability to use fuel grade alcohol is significant because fuel grade alcohol is a readily available commodity product. Additionally, ethanol is generally considered an environmentally desirable and renewable resource. Many jurisdictions offer incentives for using products like ethanol.

The reaction zone can include one or more reactors which allow sufficient mixing of the PET feed, ethanol and catalyst such as continuous stirred tank reactors, plug flow reactors, batch reactors, or combinations thereof.

The ethanolysis reaction is preferably conducted at a temperature of at least about 180°C, more preferably at least about 195°C Although lower temperatures can be used, conversion can be undesirably poor. Preferably, the reaction is conducted at a temperature no greater than about 300°C, more preferably no greater than about 250°C. Although higher temperatures can be used, such higher temperatures can lead to an undesirable amount of byproducts, for example diethyl ether.

The ethanolysis reaction can be conducted at pressures below atmospheric pressure, for example 80 kPa, or at atmospheric pressure. Preferably, the ethanolysis reaction is conducted at a pressure greater than atmospheric pressure, more preferably a pressure of at least about 200 kPa, more preferably at least about 1,000 kPa, more preferably at least about 2,000 kPa. Preferably, the ethanolysis reaction is conducted at a pressure no greater than about 6,000 kPa, more preferably no greater than about 5,000 kPa. The foregoing are examples and the pressure may vary significantly while the reaction progresses, particularly if conducting closed batch ethanolysis. For example, in a closed batch system, pressure will generally decrease as the reaction progresses. Although the pressure is somewhat dependent upon the temperature used, the wide range of conditions for which ethanol and waste PET remains in liquid phase allows the temperature and pressure to be controlled independently of the other.

The reaction product mixture is then subjected to separation to recover reaction products including ethanol, DET and ethylene glycol and, optionally, DEI, DEN and other desired components. During separation, additional components can be recovered if desired. Examples of such additional components include paraxylene, if present, other reacted and unrelated polymers or desirable compounds which may be present in the PET. As noted above, a portion of the reaction product mixture can be used as a solvent for the PET feed. In some continuous process embodiments, a portion of the reaction product mixture is removed while additional reaction components are introduced. Some portion of the reaction mixture may also be purged to maintain effective continuous operation.

Separation can be conducted by crystallization, distillation, filtration, liquid/liquid phase separation, solvent extraction or other known separation techniques or a combination of separation techniques. Preferably, separation comprises a first separation zone for recovering ethanol, a second separation zone for recovering DET and ethylene glycol and a third separation zone for recovering purified ethylene glycol. Separation can optionally include one or more purification steps for removing one or more components present with the reaction products. In one embodiment, the first and second separation zones include distillation and liquid/liquid phase separation. Liquid/liquid phase separation is not an effective separation means for a recovery of DMT from a methanolysis process because DMT typically melts at about 140-142°C and is miscible with ethylene glycol above that temperature.

Separation is preferably conducted to recover at least a first fraction comprising primarily ethanol and light reaction by-products, a second fraction comprising a major portion of ethylene glycol, a third fraction comprising primarily DET and a fourth fraction comprising high-boiling and non-volatile compounds. In a preferred embodiment, a first fraction is recovered in a first reaction zone and a second fraction, a third fraction and a fourth fraction are recovered in a second separation zone. However, fractions may be recovered in parts or a combination of fractions may be recovered together. Additionally, portions of a fraction may be recovered at different stages of the separation. For example, a portion of a first fraction comprising primarily ethanol and light by-products may be recovered at one point during separation and another portion of the first fraction may be recovered using distillation. Separation equipment may be part of more than one separation zone. In one embodiment, a portion of a first fraction comprising primarily ethanol and light by-products is recovered using a flash drum in a first separation zone and another portion of the first fraction is recovered in a distillation column which distillation column is a part of the first separation zone and part of a second separation zone.

Preferably, separation includes distillation. Distillation can be performed using one or more distillation columns as part of a first separation zone to form a first fraction comprising primarily ethanol and light reaction byproducts. Preferably, one or more distillation columns is used as part of a second separation zone such that a second fraction comprising a major portion of ethylene glycol, a third fraction comprising primarily DET and a fourth fraction comprising high-boiling compounds are recovered. In an embodiment, the first separation zone includes a distillation column which operates at or near atmospheric pressure and the second separation zone includes a distillation column operating at below atmospheric pressure. In another embodiment, separation includes a distillation column which forms at least part of a first separation zone and at least part of a second separation zone. Preferably, in such embodiment, at least a portion of a first fraction comprising primarily ethanol and light reaction byproducts, a second fraction comprising a major portion of ethylene glycol, a third fraction comprising primarily DET and a fourth fraction comprising high-boiling and non-volatile compounds are recovered from the distillation column.

All or a portion of ethanol recovered from separation can be recycled for use in the ethanolysis reaction. Such recycling can be practiced by using ethanol recovered from separation as solvent for the PET feed. Such recycling can also be practiced by introducing ethanol, recovered from separation, either upstream of the ethanolysis reaction zone or in the ethanolysis reaction zone. In one embodiment, a first fraction comprising primarily ethanol and light reaction byproducts is recovered in a first separation zone, all or part of the first fraction is treated to remove at least a portion of the light byproducts from the first fraction, preferably by condensation or other known separation techniques, and at least a portion of the ethanol of the first fraction is recycled for use in the ethanolysis reaction or as solvent for PET. Optionally, all or a portion of the first fraction may be subjected to other treatments and/or stored and/or mixed with another supply of ethanol prior to use in the ethanolysis reaction or as solvent for PET. In an embodiment, all or a portion of ethanol from a first fraction may be introduced into a reaction zone, utilizing the heat content of such ethanol from the first fraction to assist in heating PET to reaction temperature.

Ethylene glycol recovered from separation, preferably in a second fraction recovered from a second separation zone, is primarily in the form of an ethylene glycol-DET azeotrope ("EG-DET azeotrope"). Although the DET concentration in the EG-DET azeotrope varies with the separation techniques employed and operation thereof, the EG-DET azeotrope typically contains less than 10 wt% DET. At temperatures above the melting point of DET (44°C, 1 atmosphere) and below the boiling point of ethylene glycol (196-198 °C), the azeotrope separates into a first layer rich in DET and a second layer rich in ethylene glycol.

The first layer, rich in DET, can be recovered by known liquid-liquid separation techniques such as decanting and is preferably returned to separation, more preferably to the second separation zone. Optionally, the first layer can be sent directly to DET product storage. The second layer, rich in ethylene glycol, can then be subjected to purification by distillation or other means in a third separation zone where ethylene glycol is recovered and the remainder of the second layer can be returned to the separation process. If the remainder of the second layer is returned to the process, the point at which it is returned depends upon the separation method or methods used in the third separation zone. For example, if distillation is used in the third separation zone, both an ethylene glycol stream and an ethylene glycol/DET azeotrope stream will be formed, and the azeotrope stream is best combined with the second fraction of the second separation zone. If separation techniques such as filtration, crystallization or distillation are employed to recover ethylene glycol from the second layer, the second layer remainder would preferably be returned to the second separation zone. Other separation techniques, for example solvent extraction or azeotropic distillation, may require additional treatment of the second layer remainder and/or recovered ethylene glycol. The EG-DET azeotrope may also contain diethylene glycol which is primarily contained in the ethylene glycol rich layer and is preferably subjected to purification in the third separation zone. A minor portion of the diethylene glycol remains in the DET rich layer and is preferably returned to separation with the DET.

In methanolysis processes, an ethylene glycol-DMT azeotrope is typically formed which can contain about 15 wt% DMT. As noted above, liquid-liquid separation techniques are not effective for recovering DMT and recovering ethylene glycol and different techniques, typically more difficult and often more energy intensive, are used.

In one embodiment, water is used to enhance separation of the ethylene glycol-DET mixture. DET can be recovered from mixtures of ethylene glycol and DET by addition of water followed by liquid-liquid separation. Addition of water increases the concentration of DET in the first layer and decreases the concentration of DET in the second layer. A liquid-liquid separation technique, for example decanting, can be employed to recover the first layer which is preferably returned to the second separation zone, or optionally sent to DET product storage. The bulk of water employed to enhance separation is found in the second layer and can be subjected to further separation.

In another embodiment, a hydrocarbon, preferably paraxylene, is used to enhance separation. Addition of such hydrocarbon increases the concentration of DET in the first layer and decreases the concentration of DET in the second layer. If hydrocarbon is used to enhance separation, the hydrocarbon will be predominantly in the first layer and would be processed together with the first layer. In such case, additional separations can be conducted. Paraxylene is particularly advantageous because, if desired, it can remain with the DET product and used in liquid-phase oxidation reaction for the production of TA as described herein. Also, some paraxylene could be used to improve handling of the DET product by depressing the melting point of the DET. In some embodiments, water and paraxylene are both present to enhance separation.

The first layer is not necessarily the lighter layer. For example, if water is used to enhance separation, the first layer is the heavier layer. In contrast, if paraxylene is used to enhance separation, the first layer will be the lighter layer.

DET is recovered from separation, preferably as a primary portion of a third fraction from a second separation zone. Although separation is typically conducted such that the third fraction from the second separation zone comprises at least 95 wt% DET, preferably at least 97 wt% DET, it may be desirable to subject the third fraction to additional separation techniques to purify the DET, for example filtration, distillation or crystallization. For example, the recovered DET may contain minor amounts of diethylene glycol (DEG), ethylene glycol or both and may also contain water. Liquid-liquid separation techniques could be employed to purify the DET. Optionally, if water is present in the recovered DET, whether or not used to enhance liquid-liquid separation, the recovered DET may be dehydrated to remove water. For further example, PET can contain isophthalate which can be present in the PET feed and which, via ethanolysis, can form DEI. DEI, if present in the reaction product mixture would typically be recovered via separation in combination as a minor component along with DET, preferably in the third fraction. DEI can optionally be separated from DET using known separation techniques such as crystallization or distillation. However, DEI can be maintained as a part of the DET product.

The remainder of the reaction product mixture, preferably recovered as a fourth fraction from a second separation zone comprises active catalyst, reaction byproducts and other high-boiling compounds. Typically, In methanolysis processes, either one or both of the DMT and ethylene glycol products is stripped contemporaneously with the methanolysis reaction or the catalyst is deactivated to terminate the reaction to avoid undesirable reactions during separation. Advantageously, catalyst present in the reaction product mixture remainder includes active catalyst suitable for catalyzing the ethanolysis reaction. Preferably, at least a portion of the reaction product mixture remainder is recycled for use in the reaction zone. Such recycle can be practiced by adding at least a portion of the remainder to the reaction zone or upstream of the reaction zone, for example to assist in dissolving the PET. Optionally, at least a portion of the remainder may be treated to create a catalyst recycle stream with a higher concentration of catalyst and recycled for use in the reaction zone.

At any stage, the feed materials or reaction product mixture can be subject to purification to reduce unwanted contaminants. Purification can be conducted in one or more stages and may be conducted in multiple stages and on different streams. Preferably, purification is performed on the reaction product mixture and may be performed before or after any separation zone. In one embodiment, purification is performed on the reaction product mixture after a first fraction is recovered in a first separation zone. In another embodiment, purification is conducted on at least a portion of a fourth fraction recovered in a second separation zone. In another embodiment, purification is performed upon the reaction product mixture after a first fraction is recovered in a first separation zone and is also performed on at least a portion of a fourth fraction recovered in a second separation zone. Purification may include by-pass lines so that all or a portion of the purification feed can by-pass all or any portion of the purification. Such by-pass lines are particularly advantageous if a variety of waste PET is used having differing contaminants so that undesired portions of the purification can be by-passed.

Because DET has a melting point of about 44°C at 1 atmosphere, the reaction products can be retained as a melt and a number of purification techniques can be utilized effectively. Purification techniques employed will depend upon the nature of the contaminants the purification is intended to remove and include centrifugation, distillation, solvent extraction, filtration, ion exchange, adsorption or other techniques may be employed. For example, if the waste PET contains insoluble contaminants such as polyolefins, polyvinylchloride, aluminum, paper, glass, dirt, or other insoluble materials, then filtration or centrifugation would be appropriate. If it is desirable to remove soluble metals, such as antimony, that are present in the waste PET as polymerization catalysts, then processes such as ion exchange or treatment with active carbon would be appropriate. Combinations of techniques may be used for purification. Preferably, purification is performed on the reaction products at a point during the separation process.

In particular, purification using ion exchange resins can be performed upon the reaction product mixture to remove soluble metals. Ion exchange is the reversible interchange of ions between a solid (ion exchange material also referred to ion exchange resin) and a liquid or melt in which there is no permanent change in the structure of the solid. Typically, conventional ion exchange resins consist of a crosslinked polymer matrix with a relatively uniform distribution of ion-active sites throughout the structure. Generally, ion exchange materials are available as spheres or sometimes granules with a specific size and uniformity to meet the needs of a particular application. Ion exchange materials have limited thermal stability. Generally, ion exchange materials are limited to temperatures up to 150°C and often have much lower temperature limitations. Ion exchange resins suitable for use in purification are available commercially and include DOWEX resins which are suitable for removal of heavy metals including antimony. The particular ion exchange resin used will depend upon a number of factors including the nature of the undesired contaminants the ion exchange resin is intended to remove.

Soluble metals can be part of a polymer product due to use such metals as catalysts in the polymerization process. Typically, ion exchange resins are unsuitable for use in high temperature environments, such as in methanolysis processes. However, the ethanolysis reaction and reaction products can be maintained at temperatures suitable for ion exchange resins. For example, reaction product can be maintained at temperatures between 44°C and 100°C. Purification using ion exchange resins is particularly advantageous to remove soluble heavy metals such as antimony which may be present in PET feed. Additionally, purification using centrifugation is particularly advantageous to remove insoluble halogenated compounds such as polyvinylchloride. The ability to process PET feed containing soluble heavy metals and/or insoluble halogenated polymers greatly increases the scope of available PET feed materials allowing for recycle of a much wider scope of PET products than may otherwise be recyclable using methanolysis or other existing recycle methods.

Ion exchange resins can also be used to remove HCl which may be present in the reaction product mixture if polyvinylchloride is present in the waste PET feed. The ability to use ion exchange resins to remove HCl allows the use of waste PET feed having much greater concentrations of polyvinylchloride than is typically suitable for other recycle methods. In some embodiments, the invention provides a method for recycling waste PET having a PET feed having greater than 1000 ppmw polyvinylchloride (on a PET basis). In other embodiments, the invention provides a method for recycling waste PET having a PET feed having greater than 1250 ppmw or even 1500 ppmw polyvinylchloride (on a PET basis).

Suitable catalysts for the ethanolysis reaction include known transesterification catalysts. Suitable catalysts include copper acetate, zinc acetate, cobalt acetate, manganese acetate, magnesium acetate, titanium and combinations thereof. Catalyst metals are preferably in the form of acetates or, in the case of titanium, in the form of titanium(IV) isopropoxide or other organic titanates, and combinations thereof. However, it was unexpectedly discovered that impurities present in mixed PET flake, for example dyes and metallic compounds, can be effective catalysts for ethanolysis of PET. Such impurities present in PET flake which are useful for catalyzing ethanolysis are referred to herein as "catalyzing impurities." Brown flake has been found to have particularly desirable amounts and types of catalyzing impurities. In one embodiment, catalyst for the ethanolysis reaction includes catalyzing impurities. In another embodiment, at least a portion of the reaction product mixture remainder contains catalyzing impurities and is advantageously used as catalyst for the ethanolysis reaction.

Surprisingly, copper phthalocyanine can be used as a suitable catalyst for the ethanolysis reaction. If copper phthalocyanine is used as a catalyst, it is preferably present in the ethanolysis reaction at concentrations of at least about 3 ppmw (with respect to PET flake). In another embodiment, waste PET flake having brown, blue or green PET or a combination thereof is advantageously used as PET feed and at least a portion of the reaction catalyst. However, presence of water in the ethanolysis reaction, for example from using fuel grade ethanol, can decrease the effectiveness of catalyzing impurities, including copper phthalocyanine. Titanium was also found to be a desirable catalyst even with fuel grade ethanol, preferably in the form of an organic titanate such as titanium(IV) ispropoxide. Preferably, if fuel grade ethanol is used or other water component is present in the reaction zone, at least a portion of the catalyst is titanium, preferably in the form of organic titanate. If titanium is used as the catalyst, typically as an organic titanate, titanium is typically present in the reaction zone to provide from about 5 ppm titanium to about 5,000 ppm titanium based on the weight of PET in the reaction zone.

Figure 1 illustrates an embodiment of this invention where the ethanolysis reaction is conducted as a batch process and the separation process is conducted as a continuous process.

In Figure 1, waste PET is fed to a batch reactor R1 in a reaction zone that has two batch reactors operating in parallel. In Figure 1, batch reactor R1 is illustrated in the feed charging mode, while batch reactor R2 is illustrated in the product discharge mode. The PET feed is combined with ethanol from an ethanol holding vessel and ethanol from a first fraction F1 from a first separation zone. A portion of the first fraction is flashed off from reaction product mixture V1 a second portion of the first fraction is recovered from a first distillation column V4 and the remaining portion of the first fraction is recovered from a second distillation column V5. A suitable catalyst from a catalyst holding vessel is fed to batch reactor R1. The reaction can proceed in one reaction vessel as the other is being emptied and charged with feed. The ethanolysis reaction proceeds in a charged reaction vessel preferably with an initial pressure of from about 200 kPa to about 1000 kPa and at an initial temperature of from about 70 °C to about 100°C. As the reaction proceeds, the temperature of the vessel is raised to be in the range from about 180 °C to about 260 °C and the pressure increased to be from about 1500 kPa to about 5000 kPa. The reaction vessel is maintained at such pressure and temperature for from about 0.25 hours to about 5.0 hours after which time the temperature is reduced until the pressure is in the range from about 10 kPa to about 500 kPa and the reaction product mixture is fed to an intermediate holding tank V2 which is part of a first separation zone.

As illustrated in Figure 1, a portion of a first fraction F1 comprising ethanol and light by-products present in the reaction product mixture is flashed off in a flash drum V1 and ethanol is condensed into the second batch reactor R1 that is being charged or alternately returned to the ethanol holding vessel. This allows the intermediate holding tank V2 to be maintained at relatively mild conditions preferably about atmospheric pressure and about 50-100°C. Such conditions allow the reaction product mixture to be maintained in a liquid phase and there is little back reaction between DET and ethylene glycol. Such an intermediate holding tank under mild conditions would not be practicable in a methanolysis process because the conditions needed to maintain DMT in a liquid phase would also give rise to an undesirable amount of back reaction between DMT and ethylene glycol.

Referring to Figure 1, a portion of the reaction product mixture is returned to the reaction zone F4, in this case to reactor R1, the reactor that is being charged. In this embodiment, the reaction product mixture is continuously fed from the intermediate holding tank to purification V3. Purification may include by-pass lines such that all or a portion of the reaction product mixture from the intermediate holding tank can by-pass all or any portion of the purification. Examples of purification techniques which can be used for purification include but are not limited to filtration, centrifugation, ion exchange, and adsorption onto active carbon or clays. The choice of purification techniques will depend on the nature of the waste PET feed that is being used. For example, if the waste PET contains insoluble contaminants such as polyolefins, polyvinylchloride, aluminum, paper, glass, dirt, or other insoluble materials, then filtration or centrifugation would be appropriate. If it is desirable to remove soluble metals, such as antimony, that are present as polymerization catalysts in the waste PET, then techniques such as ion exchange or treatment with active carbon would be appropriate. Combinations of techniques may be used.

In Figure 1, reaction product mixture from purification V3 is fed to a first distillation column V4 which is part of the first separation zone. The first distillation V4 column is operated at or near atmospheric pressure. A second portion of the first fraction F1 comprising primarily ethanol and light reaction by-products is recovered from the first distillation column V4.

In Figure 1, after the first distillation column V4, the reaction product mixture is fed to a second distillation column V5 which forms part of the first separation zone and part of a second separation zone. The second distillation column V5 is operated at less than atmospheric pressure. Four fractions are recovered from the second distillation column V5: the remaining portion of the first fraction F1 comprising primarily ethanol and light reaction by-products, a second fraction F2 comprising a major portion of ethylene glycol, a third fraction F3 comprising primarily DET and a fourth fraction F4 comprising high-boiling compounds. The remainder of the first fraction is combined with the other portions of the first fraction and the first fraction is fed to a condenser and condensed ethanol is returned to the ethanol holding vessel and the remainder of the first fraction is purged.

As seen in Figure 1, the second fraction F2 is sent to a decanting tank V6 for liquid-liquid separation where the second fraction F2 forms a first layer L1 rich in DET and a second layer L2 rich in ethylene glycol. Water from a holding vessel V7 is added to the second fraction F2 to increase the concentration of DET in the first layer L1 and decrease the concentration of DET in the second layer L2. Liquid from the first layer L1 is returned to the second distillation column V5 or alternatively to the DET product storage tank V9 or both and liquid from the second layer L2 is sent to a third separation zone V8. The third fraction F3 is sent to a DET product holding tank V9. A portion of the fourth fraction F4 is recycled for use in the reaction zone and the remainder of the fourth fraction is purged.

As shown in Figure 1, the second layer L2 is sent to the third separation zone V8 from which ethylene glycol is recovered and sent to an ethylene glycol product holding tank V10.

The resulting ethyl ester product can then be converted to a carboxylic acid product. Preferably, the ethyl ester product is an aromatic ethyl ester, more preferably a aromatic diethyl ester. The ethyl ester can be oxidized by reacting the ethyl ester with oxygen to form the corresponding carboxylic acid and acetic acid.

As used herein, "aromatic hydrocarbon" means a molecule composed of carbon atoms and hydrogen atoms, and having one or more aromatic ring, for example a benzene or naphthalene ring. For purposes of this application, "aromatic hydrocarbon" includes such molecules having one or more hetero atoms such as oxygen or nitrogen atoms. "Methyl aromatic hydrocarbon" means an aromatic hydrocarbon molecule having one or more methyl groups attached to one or more aromatic rings. "Aromatic ethyl esters" means the ethyl esters of aromatic acids having one or more ethyl groups. As used herein, "aromatic carboxylic acid" means an aromatic acid having one or more carboxylic acid groups.

We have found that aromatic ethyl esters are useful as feedstock or feedstock components for the production of aromatic carboxylic acids. In one embodiment, this invention provide feedstocks useful for the production of aromatic carboxylic acids. Such feedstocks include one or more aromatic ethyl esters. Aromatic ethyl esters can be used alone as such feedstock. In a preferred embodiment, one or more aromatic ethyl esters are used as a component of a feedstock for the production of aromatic carboxylic acids. Aromatic ethyl esters are particularly useful as feedstock for liquid-phase oxidation processes to produce aromatic carboxylic acids.

Aromatic carboxylic acids for which the invention is suited include carboxylated species having one or more aromatic rings and which can be manufactured by reaction of gaseous and liquid reactants in a liquid phase system. Examples of aromatic carboxylic acids for which the invention is particularly suited include terephthalic acid, phthalic acid, isophthalic acid, trimellitic acid and naphthalene dicarboxylic acids.

Feedstocks in accordance with this invention comprise one or more aromatic ethyl ester. The particular aromatic ethyl ester or combination or aromatic ethyl esters used will depend upon the desired aromatic carboxylic acids. For a particular desired aromatic carboxylic acid, the corresponding aromatic ethyl ester precursor is used as all or a component of the feedstock. For example, for terephthalic acid, diethyl terephthalate is used as all or a portion of the feedstock. For isophthalic or phthalic acids, diethyl isophthalate or diethyl phthalate, respectively, is used as all or a component of the feedstock. In one embodiment, more than one aromatic ethyl ester is used all or components of the feedstock which can optionally be used to produce more than one aromatic carboxylic acid.

In one embodiment, a feedstock useful for the production of aromatic carboxylic acid includes at least one aromatic ethyl ester component and at least one methyl aromatic hydrocarbon component. For example, for production of terephthalic acid, the feedstock can include paraxylene and diethyl terephthalate. As a further example, for the co-production of terephthalic acid and isophthalic acid, the feedstock preferably includes paraxylene, diethyl terephthalate and one or both of metaxylene and diethyl isophthalate. For the production of naphthalene dicarboxylic acids, a preferred feedstock includes at least one diethyl naphthalate component and at least one dimethyl naphthalene component.

Proportions of feedstock components are not critical to the invention. However, it is preferred that feedstock comprise at least 1 wt% aromatic ethyl ester components (measured on the basis of total aromatic carboxylic acid precursors for the desired aromatic carboxylic acid or acids). More preferably the feedstock comprises at least 5 wt% aromatic ethyl ester components, more preferably at least 10 wt% aromatic ethyl ester components. Although the feedstock can comprise up to 100 wt% aromatic ethyl ester components (measured on the basis of total aromatic carboxylic acid precursors for the desired aromatic carboxylic acid or acids), preferably the feedstock comprises less than 100 wt% aromatic ester compounds. The feedstock can contain significantly less than 100 wt% aromatic ester compounds, for example less than 50 wt% or even less than 30 wt% aromatic ethyl ester components. Optionally, the proportion of aromatic ethyl esters is selected and or adjusted to maintain a desired level and composition of solvent in the reaction zone.

For manufacture of aromatic carboxylic acids, it is preferred to use relatively pure feed materials, and more preferably, feed materials in which the total content of the feed components (including all precursors corresponding to the desired acid or acids) is at least about 95 wt.%, and more preferably at least 98 wt.% or even higher.

The liquid-phase oxidation of aromatic ethyl esters to produce aromatic carboxylic acids can be conducted as a batch process, a continuous process, or a semi-continuous process. The oxidation reaction takes place in a reaction zone which can comprise one or more reactors. The reaction zone can include mixing vessels or conduits where components are combined and oxidation reactions occur. A reaction mixture is formed by combining components comprising feedstock, solvent, and catalyst optionally with a promoter, typically bromine. In a continuous or semi-continuous process, the reaction mixture components preferably are combined in a mixing vessel before being introduced into an oxidation reactor, however, the reaction mixture can be formed in the oxidation reactor.

Solvents comprising an aqueous carboxylic acid, for example benzoic acid, and especially a lower alkyl (e.g., C₁-C₈) monocarboxylic acid, for example acetic acid, are preferred because they tend to be only sparingly prone to oxidation under typical oxidation reaction conditions used for manufacture of aromatic carboxylic acids, and can enhance catalytic effects in the oxidation. Specific examples of suitable carboxylic acid solvents include acetic acid, propionic acid, butyric acid, benzoic acid and mixtures thereof. Ethanol and other co-solvent materials which oxidize to monocarboxylic acids under the oxidation reaction conditions also can be used as is or in combination with carboxylic acids with good results. Of course, for purposes of overall process efficiency and minimizing separations, it is preferred that when using a solvent comprising a mixture of monocarboxylic acid and such a co-solvent, the co-solvent should be oxidizable to the monocarboxylic acid with which it is used.

Typically, a portion of the solvent in the reaction zone is lost due to either solvent burning (oxidation) or through process losses including recovery inefficiencies. In some commercial operations, such losses can be as high as 2 wt% of the solvent or even 4wt% or higher. Because of such losses, additional solvent, typically referred to as make-up solvent, is added to the process to make up for solvent loss. This invention can provide additional benefit if the solvent comprises acetic acid because the oxidation of aromatic ethyl esters produces acetic acid. In cases where the solvent comprises acetic acid, use of aromatic ethyl esters can reduce or even eliminate the amount of make-up solvent used. In one embodiment, the proportion of aromatic ethyl ester components in the feedstock is selected on the basis of the amount of acetic acid added to the reaction zone by oxidation of the aromatic ethyl ester components so as to achieve or approach a desired reduction in the amount of make-up acetic acid employed.

Catalysts used according to the invention comprise materials that are effective to catalyze oxidation of the aromatic ethyl ester feed to aromatic carboxylic acid. Preferably, the catalyst is soluble in the liquid oxidation reaction body to promote contact among catalyst, oxygen and liquid feed; however, heterogeneous catalyst or catalyst components may also be used. The catalyst comprises at least one suitable heavy metal component such as a metal with atomic weight in the range of about 23 to about 178. Examples of suitable heavy metals include cobalt, manganese, vanadium, molybdenum, chromium, iron, nickel, zirconium, hafnium or a lanthanoid metal such as cerium. Suitable forms of these metals include for example, acetates, hydroxides, and carbonates. The catalyst preferably comprises cobalt compounds alone or in combination with one or more of manganese compounds, cerium compounds, zirconium compounds, or hafnium compounds.

Typically, the catalyst can comprise a promoter which is used to promote oxidation activity of the catalyst metal, preferably without generation of undesirable types or levels of by-products, and is preferably used in a form that is soluble in the liquid reaction mixture. Halogen compounds are commonly used as a promoter, for example hydrogen halides, sodium halides, potassium halides, ammonium halides, halogen-substituted hydrocarbons, halogen-substituted carboxylic acids and other halogenated compounds. Preferably, bromine compounds are used as a promoter. Suitable bromine promoters include bromoanthracenes, Br₂, HBr, NaBr, KBr, NH4Br, benzyl-bromide, bromo acetic acid, dibromo acetic acid, tetrabromoethane, ethylene dibromide, bromoacetyl bromide or mixtures thereof.

The oxidation reaction is conducted in a reaction zone comprising at least one oxidation reactor. The oxidation reactor can comprise one or more reactor vessels. Suitable oxidation reactors are those which allow for mixing of liquid and gaseous reactants and venting of gaseous product for controlling the heat of the reaction. Reactor types which can be used include, but are not limited to, continuous stirred tank reactors and plug-flow reactors. Commonly, oxidation reactors comprise a columnar vessel having one or more mixing features for distributing oxygen within a liquid phase boiling reaction mix. Typically, the mixing feature comprises one or more impellers mounted on a rotatable or otherwise movable shaft. For example, impellors may extend from a rotatable central vertical shaft Reactors may be constructed of materials designed to withstand the particular temperatures, pressures and reaction compounds used. Generally, suitable oxidation reactors are constructed using inert materials such as titanium or may be lined with materials such as titanium or glass to improve resistance to corrosion and other deleterious effects. For example, titanium and glass, or other suitable corrosion resistant material would typically be used for reactors and some other process equipment for the production of terephthalic acid from diethyl terephthalate, and optionally paraxylene, using a solvent comprising acetic acid and a catalyst system which can include a bromine promoter under typical reaction conditions due to corrosivity of the acid solvent and certain reaction products, for example methyl bromide.

A source of molecular oxygen is also introduced into the reaction zone, preferably into the oxidation reactor. Typically, an oxidant gas is used as a gaseous source of molecular oxygen. Air is conveniently used as a source of molecular oxygen. Oxygen-enriched air, pure oxygen and other gaseous mixtures comprising molecular oxygen, typically at least about 10 vol.%, also are useful. As will be appreciated, as molecular oxygen content of the source increases, compressor requirements and handling of inert gases in reactor off-gases are reduced. The source of molecular oxygen may be introduced into the reaction zone in one or more locations and is typically introduced in such a manner as to promote contact between the molecular oxygen and the other reaction compounds. Commonly, an oxidant gas is introduced in the lower portion of a reactor and is distributed by mixing features such as one or more impellors mounted on a rotating shaft. Molecular oxygen content of oxidant gas varies but typically will range from about 5 to about 100 vol% molecular oxygen. To avoid the formation of potentially explosive mixtures, oxidant gas is generally added such that unreacted oxygen in the vapor space above the liquid reaction is below the flammable limit. Keeping oxygen content of the off-gas below the flammable limit depends upon the manner and rate of oxygen introduction, reaction rate (which is impacted by reaction conditions) and off-gas withdrawal. Typically, oxidant gas is supplied in an amount in relation to such operating parameters such that the reactor overhead vapor contains about 0.5 to about 8 vol.% oxygen (measured on a solvent-free basis).

Proportions of feed, catalyst, oxygen and solvent are not critical to the invention and vary not only with choice of feed materials and intended product but also choice of process equipment and operating factors. Solvent to feed weight ratios suitably range from about 1:1 to about 30:1. Oxidant gas typically is used in at least a stoichiometric amount based on feed but not so great that unreacted oxygen in the vapor space above the liquid reaction would exceed the flammable limit. Advantageously, the oxidation of aromatic ethyl esters to aromatic carboxylic acids has a lower stoichiometric requirement for oxygen than oxidation of methyl aromatic hydrocarbons to form aromatic carboxylic acids. For example, the oxidation of one mol dimethyl aromatic hydrocarbons to one mol of the corresponding aromatic dicarboxylic acids consumes a minimum of 3 mols of O₂ and produces two mols of H₂O. The H₂O by-product is often undesirable and additional processing must be conducted to remove this by-product from the other solvent components prior to recycle. In contrast the required stoichiometric amount of O₂ for the oxidation of one mol aromatic diethyl esters to one mol of the corresponding aromatic dicarboxylic acid is only 2 mols of O₂ and the by-product of the oxidation is acetic acid which can be used as solvent and thus may not require removal. Although oxygen is typically provided to the reaction zone in greater than stoichiometric amount, use of aromatic ethyl ester components in place of all or a portion of methyl aromatic hydrocarbon feed components reduces the overall oxygen demand for production of a desired amount of aromatic carboxylic acid. In cases where production rate of aromatic carboxylic acid is limited by oxygen demand, use of aromatic ethyl esters in place of methyl aromatic hydrocarbons can lead to an increase in production rate of aromatic carboxylic acids.

Catalysts suitably are used in concentrations of catalyst metal, based on weight of aromatic hydrocarbon feed and solvent, greater than about 100 ppmw, preferably greater than about 500 ppmw, and less than about 10,000 ppmw, preferably, less than about 7,000 ppmw, more preferably less than about 5000 ppmw. Preferably a halogen promoter, more preferably bromine, is present in an amount such that the atom ratio of halogen to catalyst metal suitably is greater than about 0.1:1, preferably greater than about 0.2:1 and suitably is less than about 4:1, preferably less than about 1:1. The atom ratio of halogen to catalyst metal most preferably ranges from about 0.25:1 to about 1:1.

Oxidation of aromatic ethyl ester to produce aromatic carboxylic acid is conducted under oxidation reaction conditions. The reaction is operated at temperatures sufficient to drive the oxidation reaction and provide desirable purity while limiting solvent burning. Heat generated by oxidation is dissipated to maintain reaction conditions. Typically, heat of reaction is dissipated by boiling the reaction mixture and removing vapors resulting from boiling from the reaction zone. Generally suitable temperatures are in excess of about 120°C, preferably in excess of 140°C, and less than about 250°C preferably less than about 230°C. Reaction temperatures of between about 145°C to about 230°C are preferred for the production of some aromatic carboxylic acids, for example, terephthalic acid and naphthalene dicarboxylic acid. At temperatures lower than about 120°C the oxidation reaction typically proceeds too slowly and results in insufficient product purity and undesirably low conversion. For example, oxidation of DET to produce terephthalic acid at a temperature less than about 120 °C can take more than 4 hours to proceed to substantial completion. The resultant terephthalic acid product may require significant additional processing due to its high level of impurities. At temperatures above 250°C, significant loss of solvent can occur due to solvent burning.

Pressure in the reaction vessel is at least high enough to maintain a substantial liquid phase comprising feed and solvent in the vessel. Generally, pressures of about 5 to about 40 kg/cm2 gauge are suitable, with preferred pressures for particular processes varying with feed and solvent compositions, temperatures and other factors but typically between about 10 to about 30 kg/cm2- Residence times in the reaction vessel can be varied as appropriate for given throughputs and conditions, with about 20 to about 150 minutes being generally suited to a range of processes. In processes, such as oxidation of aromatic diethyl esters to terephthalic or isophthalic acids using acetic acid and water as solvent for the reaction mixture, solids contents can be as high as about 50 wt.% of the liquid reaction body, with levels of about 10 to about 35 wt.% being more typical. As will be appreciated by those skilled in the manufacture of aromatic acids, preferred conditions and operating parameters vary with different products and processes and can vary within or even beyond the ranges specified above.

The reactor overhead vapor typically comprises solvent and, if methyl aromatic hydrocarbons are present, water. Advantageously, substitution of aromatic ethyl ester components for all or a portion of the methyl aromatic hydrocarbon components in the feedstock reduces the production of excess water thereby reducing the need to treat or otherwise use or dispose of excess water. For example, the liquid phase oxidation of paraxylene to form terephthalic acid produces about 2 moles of excess water per mole of terephthalic acid produced. In contrast, the liquid phase oxidation of DET to form terephthalic acid can result in production of little or no excess water. The overhead gas also may contain unreacted oxidant gas, unreacted feedstock components, gaseous reaction byproducts, such as carbon oxides, vaporized reaction by-products such as methyl bromide,, catalyst, or a combination thereof. If air is used as the oxidant gas, then the reactor overhead vapor typically comprises solvent, water, unreacted feedstock components, mono-ethyl aromatic hydrocarbons, excess oxygen (if any), carbon oxides, nitrogen gas and reaction by-products.

Optionally, reactor overhead vapor can be processed to return recyclable components to the reaction zone. Typically, the reactor overhead vapor is at high pressure and temperature and energy can be recovered from the reaction overhead vapor, preferably after treatment of the vapor to return solvent and unreacted feedstock components to the reaction zone. Such treatments can include a high efficiency separation, for example as described in U.S. Patent 5,723,656 to Abrams which is incorporated by reference herein. Such high efficiency separation helps reduce solvent loss and helps reduce the amount of make-up solvent used in the reaction by returning reaction solvent (excluding water) and unreacted aromatic ethyl esters to the reaction zone. High efficiency separation also allows substantial retention of water in a gaseous phase useful for energy recovery.

Energy can be recovered in the form of heat through heat exchange with another material, for example water to produce steam, which material can then be used in other parts of the process, for other processes or both. Energy can also be recovered in the form of work, for example using an expander or other device capable of converting work into energy. Energy can be recovered in the form of heat and in the form of work either in series or parallel. Recovered energy can be used to offset the energy requirements of the process, used in other processes, stored, returned to an energy grid, any combination of uses or any other desired use.

Depending on the specific catalyst components, feedstock and solvent used, reactor overhead vapor may contain corrosive compounds or other compounds detrimental to equipment used for energy recovery. For example, if bromine is used as a promoter in the liquid phase oxidation of DET to produce terephthalic acid, methyl bromide may be present in the reactor overhead vapor

Other treatments or a combination of treatments can be used on the reactor overhead vapor. For example, the reactor overhead vapor, preferably after other treatment to recover solvent and unreacted feedstock components, can be treated for removing corrosive or combustible materials. Although any treatment for removing corrosive or combustible materials can be used, preferably without significant condensation of liquid water, preferably the reactor overhead vapor is subjected to a thermal oxidation process, more preferably a catalytic thermal oxidation process. Preferably, treated reactor overhead vapor is directed to a catalytic oxidation apparatus wherein the treated reactor overhead vapor is contacted with a suitable catalytic material at high temperature and pressure in the presence of air or other source of molecular oxygen and the corrosive and combustible byproducts are catalytically oxidized into less corrosive or more environmentally compatible materials. Optionally, preheating can be employed before such catalytic oxidation treatment. Preheating can be accomplished by any suitable means such as a heat exchanger, direct steam injection or other means known in the art.

Such catalytic oxidation treatment can be used to reduce or eliminate corrosive alkyl bromide compounds. Additionally, such catalytic oxidation treatment can remove residual solvent which may be present. Preferably, the reactor overhead vapor has been treated to remove a substantial portion of the solvent so that the load on the catalytic oxidation unit is reduced. A high level of reaction solvent in the stream directed to catalytic oxidation treatment would result in an unacceptably large temperature rise in the catalytic oxidation unit. Furthermore, the combustion of reaction solvent that otherwise could be recycled to oxidation would be an economic loss.

Oxidation catalysts for such catalytic oxidation are commercially available from, for example, Engelhard Corp. or AlliedSignal Inc. Typically, such oxidation catalysts comprise the transition group elements of the Periodic Table (IUPAC), for example the Group VIII metals. Platinum is a preferred metal for catalytic oxidation treatment. Such catalyst metals may be used in composite forms such as oxides. Typically, the support for such catalyst metals may be less catalytically active or inert. The support can be present in a composite. Typical catalyst support materials include mullite, spinel, sand, silica, alumina, silica alumina, titania, zirconia, alpha alumina, gamma alumina, delta alumina, eta alumina, and composites of the foregoing. Such catalytic oxidation catalysts can be used in any convenient configuration, shape or size which exposes the oxidation promoting components to stream being subjected to catalytic oxidation. For example, the catalyst can be in the form of pellets, granules, rings, spheres, etc.

Other optional treatments for the reactor overhead vapor include scrubbing to remove acidic, inorganic materials such as bromine or hydrogen bromide. Bromine and hydrogen bromide are produced by the catalytic oxidation of alkyl bromides and organic impurities.

In a particular embodiment, the invention is used for the boiling liquid phase oxidation of a feedstock comprising DET and paraxylene to terephthalic acid. Optionally, the feedstock also comprises DEI and/or metaxylene for co-production of terephthalic acid and isophthalic acid. The feedstock and solvent are continuously introduced into a reaction zone comprising a reaction vessel. Catalyst and promoter, each preferably also dissolved in solvent, are introduced into the reaction vessel. Acetic acid or aqueous acetic acid is a preferred solvent, with a solvent to feed ratio of about 2:1 to about 5:1 being preferred. The catalyst preferably comprises cobalt in combination with manganese, cerium, zirconium, hafnium, or any combination thereof and a bromine source. The catalyst is suitably present in amounts providing about 600 ppmw to about 3500 ppmw of catalyst metals based on weight of the aromatic hydrocarbon and solvent. The promoter most preferably is present in an amount such that the atom ratio of bromine to catalyst metal is about 0.2:1 to about 1.5:1. Oxidant gas, which is most preferably air, is supplied to the reactor vessel at a rate effective to provide at least about 3 to about 5.6 moles molecular oxygen per mole of aromatic hydrocarbon in the feedstock so that the reactor overhead vapor contains from about 0.5 to about 8 vol.% oxygen (measured on a solvent-free basis).

In such particular embodiment, the reaction vessel is preferably maintained at about 150 to about 225°C under pressure of about 5 to about 40 kg/cm² gauge. Under such conditions, contact of the oxygen and feedstock components in the liquid body results in formation of solid terephthalic acid crystals, typically in finely divided form. Under such conditions, contact of the oxygen and diethyl hydrocarbon components in the liquid body results in the formation of acetic acid and solid terephthalic acid crystals. Solids content of the boiling liquid slurry typically ranges up to about 40 wt.% and preferably from about 20 to about 35 wt.%, and water content typically is about 5 to about 20 wt.% based on solvent weight. Boiling of the liquid body for control of the reaction exotherm causes volatilizable components of the liquid body, including solvent and water of reaction, to vaporize within the liquid along with vaporized byproducts, unreacted feedstock components. Unreacted oxygen and vaporized liquid components escape from the liquid into the reactor space above the liquid. Other species, for example nitrogen and other inert gases that are present if air is used as an oxidant gas, carbon oxides, and vaporized by-products, e.g., , methyl acetate and methyl bromide, also may be present in the reactor overhead vapor.

In such embodiment, aromatic dicarboxylic acid reaction product, slurried or dissolved in a portion of the liquid body, is removed from the vessel. The product stream can be treated using conventional techniques to separate its components and to recover the aromatic carboxylic acid contained therein, usually by crystallization, liquid-solid separations and drying. Conveniently, a slurry of solid product in the liquid is centrifuged, filtered or both, in one or more stages. Soluble product dissolved in the liquid can be recovered by crystallization. Liquid comprising water, solvent, unreacted feed material, and often also containing one or more liquid catalyst, promoter and reaction intermediates, can be returned to the reaction vessel, The production of terephthalic acid from DET may progress more slowly than the conversion of paraxylene to terephthalic acid. However, unreacted DET which leaves the reaction zone either with the reactor overhead vapor or with the product can be recovered with solvent and returned to the reaction zone and so the effective residence time of the DET is increased to permit the slower reaction to progress effectively.

In such embodiment, aromatic dicarboxylic acid product recovered from the liquid can be used or stored as is, or it may be subjected to purification or other processing. Purification is beneficial for removing by-products and impurities that may be present with the aromatic dicarboxylic acid that is recovered. For aromatic dicarboxylic acids such as terephthalic and isophthalic acids, purification preferably involves hydrogenation of the oxidation product, typically dissolved in water or other aqueous solvent, at elevated temperature and pressure in the presence of a catalyst comprising a metal with hydrogenation catalytic activity, such as ruthenium, rhodium, platinum or palladium, which typically is supported on carbon, titania or other suitable, chemically-resistant supports or carriers for the catalyst metal. Purification processes are known, for example, from US 3,584,039, US 4,782,181, 4,626,598 and US 4,892,972.

Advantageously, use of aromatic ethyl esters can reduce the formation of some impurities. For example, a significant impurity in crude terephthalic acid (produced from paraxylene) is 4-carboxybenzaldehyde (4-CBA) which is an intermediate in the formation of terephthalic acid from paraxylene. Often, significant effort is expended to reduce the amount of 4-CBA present in terephthalic acid. In contrast, 4-CBA is not an intermediate of the formation of terephthalic acid from DET. DET could be used in a feedstock to help reduce the formation of 4-CBA in the terephthalic acid product.

If purification is conducted with water as solvent, washing with water to remove residual oxidation solvent from the solid aromatic carboxylic acid can be carried out as an alternative to drying. Such washing can be accomplished using suitable solvent exchange devices, such as filters, as disclosed in US 5,679,846, and US 5,175,355- Optionally, all or a portion of mother liquor from purification processes may be sent, directly or indirectly, to a high efficiency separation apparatus or other treatment. For example, if one or more high efficiency distillation columns are used to perform the high efficiency separation, all or a portion of the purification mother liquor can be used as reflux for one or more of such high efficiency distillation columns.

Typically, oxidation mother liquor is separated from the unpurified aromatic carboxylic acid product through separation techniques known in the art, for example, filtration, centrifuge, or combinations of known methods. It is preferable to recycle at least a portion of the mother liquor and commercial operations typically recycle a significant portion of the mother liquor. For example, such mother liquor can be recycled directly or indirectly to the oxidation reactor or the high efficiency separation apparatus. Such recycle is particularly desirable in the production of terephthalic acid from a feedstock comprising DET and paraxylene. Mother liquor can be separated from purified aromatic dicarboxylic acid product through similar techniques and such mother liquor may be recycled, with or without treatment, for use in other stages of this process or in other processes.

It is understood that reaction by-products may be formed during the reaction, for example aromatic mono-ethyl esters. Some by-products will enter the vapor phase and be treated as part of the reactor overhead vapor, some by-products will remain with the oxidation mother liquor and some by-products will be present with aromatic carboxylic acid product. The same by-product may be present in more than one of these streams. Such by-products or portions thereof can be recovered and, if desired, recycled to the reaction zone or purged either after recovery or as part of a purge stream. Preferably, by-products which can be oxidized to form either aromatic carboxylic acids or solvent are recycled to the reaction zone.

In addition to use for producing aromatic carboxylic acids, aromatic diethyl esters can also be used in oxidation processes to produce excess acetic acid which can be recovered and sold or used in other processes. Acetic acid is a highly desired commodity and the ability to produce it as a co-product could be particularly advantageous. In one embodiment, this invention provides a method of producing acetic acid either to reduce solvent losses or to produce excess acetic acid. In such embodiment, aromatic ethyl esters are used in liquid phase oxidation process of the kind herein described.

Aromatic carboxylic acids can be used to form polymers. Although numerous ways exist to form polymers from carboxylic acids, typically, carboxylic acids can be used in a condensation reaction with ethylene glycol to form an aromatic ester-ethylene molecule and subsequently polymerized. For example, terephthalic acid can be reacted with ethylene glycol to form PET. For further example, naphthalene dicarboxylic acid can be reacted with ethylene glycol to form PEN. Typically, condensation reactions are performed under heat and in the presence of an acid catalyst. Water, formed as a byproduct is removed from the reaction, for example through distillation, to drive the reaction and minimize back-reaction.

In one embodiment, PET is formed from terephthalic acid and ethylene glycol. In a first stage of the reaction, an ester is formed between from the acid and two molecules of ethylene glycol. In a second stage, ester is heated to a temperature in the range from about 210 to about 290°C and at a low pressure. A number of catalysts are known to catalyze the polymerization reaction which can be used. Preferably, the catalyst includes antimony compounds for example antimony(III) oxide. In this second stage, PET is formed and a portion of the ethylene glycol is regenerated. The ethylene glycol is typically removed and recycled.

In another embodiment, at least a portion of the ethylene glycol used to form the polymer was formed in the methanolysis reaction.

Alternatively, an aromatic carboxylic acid can be converted to a methyl ester and reacted with ethylene glycol in an alcoholic transesterification reaction to form an aromatic ester-ethylene molecule which is then polymerized. In such a reaction, methanol is produced as a by-product and is removed to drive the reaction forward.

The aromatic ester-ethylene molecules are optionally purified either prior to being polymerized or between stages of staged polymerization or both. Additionally, other monomers or oligomers may be introduced into the polymerization process to produce copolymer, terpolymers, etc.

The invention has been described above and in examples below by reference to specific embodiments, but it will be understood that changes can be made to the apparatus and process specifically described which are yet within the scope of the invention. For example, additional apparatuses can be included, such as heat exchangers, preheaters, additional condensers, reboilers, energy recovery devices, and other equipment used in commercial operations without departing from the scope of the invention. As further example, additional steps such as treatment of various streams to remove impurities or to alter the physical or chemical properties of streams may be practiced without departing from the scope of the invention.

The non-limiting examples below further illustrate various aspects of embodiments of the invention.

For Examples 1-5, Unless otherwise indicated, the ethanolysis reaction in the examples below was conducted using a 2 Liter Parr Reactor. Reactants were placed in the reactor, the reactor was sealed and the atmosphere in the reactor was purged with nitrogen. Unless otherwise noted, the reactor was initially pressurized to 40 psig (approx. 276 kPa), the stirrer activated and the reactor brought to 200°C for 2 hours. After 2 hours, heat was turned off and the reactor was allowed to cool ambient temperature overnight (with continued stirring). Afterwards, the stirring was stopped and the reaction products were separated using distillation. Ethanol was recovered using stirred distillation at ambient pressure and the remaining reaction product was subjected to vacuum distillation. Vacuum distillation was conducted at from about 27"-29" Hg.

### EXAMPLE 1

300 g of PET flake of the type indicated in Table 1 was reacted with ethanol at an ethanol:PET weight ratio of 3:1 in accordance with the above procedure. The ethanol had a water content of 0.0734 wt%. No external catalyst was added. Both mixed flake and the clean clear flake were obtained through NAPCOR, the National Association for PET Container Resources The mixed flake contained about 55 wt% brown flake with the rest being primarily green, amber and clear PET flake. The virgin bottle resin was obtained from Wellman Inc. as product number 61802. The theoretical maximum percentages of DET and ethylene glycol in the reaction mixture were 28.86 wt% and 8.06 wt%, respectively.

| TABLE 1 DET and Ethylene Glycol Recovered using Various PET Flake | | | |
|---|---|---|---|
| Run # | Flake Used | DET Recovered | Ethylene Glycol Recovered |
| 1 | Mixed | 22.3 wt% | 6.25 wt% |
| 2 | Mixed | 25.2 wt% | 6.54 wt% |
| 3 | Virgin Bottle Resin | 0.53 wt% | 0.21 wt% |
| 4 | Clean Clear | 1.06 wt% | 0.45 wt% |
| 5 | Brown | 24.16 wt% | 5.41 wt% |
| 6 | Amber | 0.80 wt% | 0.37 wt% |
| 7 | Mixed w/o Brown | 2.8 wt% | 0.51 wt% |
| 8 | Clean Clear w/ Brown* | 25.21 wt% | 6.0 wt% |

| | | | |
|---|---|---|---|
| * 45wt% Clean Clear Flake and 55% Brown | | | |

The results from Runs 1 and 2 in Table 1 demonstrated that even without any added catalyst, mixed flake contained catalyzing impurities that catalyzed the ethanolysis reaction. Runs 3 through 8 revealed that the catalyzing impurities were primarily present in the brown flake. We surprisingly discovered that copper phthalocyanine, a pigment commonly used in brown PET, is a particularly effective catalyst for ethanolysis reaction.

### EXAMPLE 2

300g of clean clear PET flake was reacted with 900g of ethanol in accordance with the procedure outlined above in the presence of titanium in the form of an organic titanate. TYZOR TPT, an organic titanate available commercially from DuPont, was used as the source of titanium. The ethanol had a water concentration of 0.0734 wt%. Organic titanate was added in an amount equal to 1000 ppmw (on a PET basis) titanium. The results are reflected as Run 9 in Table 2 below. Run 10 was conducted in accordance with the above procedure using 200g clean clear PET flake and 600g ethanol. Organic titanate was added in an amount equal to 17.6 ppmw titanium (on a PET basis). The results are reflected in Table 2 below.

| TABLE 2 DET and Ethylene Glycol Recovered using Organic Titanate | | | |
|---|---|---|---|
| Run # | Titanium | DET Recovered | Ethylene Glycol Recovered |
| 9 | 1000 ppmw | 25.61 wt% | 6.24 wt% |
| 10 | 17.6 ppmw | 25.19 wt% | 6.36 wt% |

Table 2 illustrates the effectiveness of organic titanate in catalyzing the ethanolysis of PET. Even the very small amount used in Run 10 was effective.

### EXAMPLE 3

Ethanolysis was conducted according to the procedure above with no added catalyst and using mixed flake PET as described in Example 1 and ethanol having 0.0734 wt% water content. Ethanol : PET ratio was 3:1 and no external catalyst was added. After distilling the reaction product as described above, the distillation bottoms were used as catalyst for further ethanolysis reactions. Additional ethanolysis reaction was conducted using 600g ethanol having 0.0734 wt% water content, 162g clean clear PET flake and 38g distillation bottoms. No additional catalyst was used. The result is illustrated in Table 3 below.

| TABLE 3 Distillation Bottoms as Ethanolysis Catalyst | | | |
|---|---|---|---|
| Run # | PET | DET Recovered | Ethylene Glycol Recovered |
| 11 | 162g clean clear 38g distillation bottoms | 24.3 wt% | 5.51 wt% |

Table 3 shows that catalyzing impurities present in the mixed flake feed remained active through the distillation process and recycle of a portion of distillation bottoms can be used to effectively catalyze the methanolysis of PET. A comparison between Run 4 in Table 1 and Run 11 in Table 3 particularly highlights the effectiveness of distillation bottoms in catalyzing the ethanolysis reaction.

### EXAMPLE 4

Additional tests were conducted to examine the effect of water on the ethanolysis reaction. The ethanolysis reaction was performed in accordance with the procedure above and the results are shown in Table 4 below. Table 4 lists the water concentration (wt%) in the ethanol used. For Runs 12-17, mixed flake (as described above) was used as the PET source and combined with ethanol in a ethanol : PET ratio of 3:1. For Run 17, the 300g of mixed flake was dried in a vacuum oven thereby removing about 1.78g of water. For Run 18, clean clear flake was used as PET feed and 20 ppmw titanium (on a PET basis) in the form of organic titanate wad added.

| TABLE 4 Effect of Water Concentration in Ethanol | | | |
|---|---|---|---|
| Run # | Water in Ethanol | DET Recovered | Ethylene Glycol Recovered |
| 12 | 0.0734 wt% | 25.2 wt% | 6.54 wt% |
| 13 | 6.98 wt% | 0.05 wt% | 0 wt% |
| 14 | 1.06 wt% | 2,8 wt% | 0.95 wt% |
| 15 | 0.50 wt% | 10.09 wt% | 2.16 wt% |
| 16 | 0.29 wt% | 16.4 wt% | 4.46 wt% |
| 17 | 0.29 wt% | 25.0 wt% | 6.1 wt% |
| 18 | 1.06 wt% | 21.4 wt% | 5.99 wt% |

The results in Table 4 illustrate that the effectiveness of catalyzing impurities in catalyzing ethanolysis of PET is sensitive to the presence of water. Even about 1 wt% water present in fuel grade ethanol significantly degraded the effectiveness of catalyzing impurities. Surprisingly, however, the organic titanate was an effective catalyst even using fuel grade ethanol (about 1 wt% water). The ability to use fuel grade ethanol is particularly significant because fuel grade ethanol is a readily obtainable commodity in many regions. Additionally, because of ethanol's affinity for water, the ability to tolerate some water in the ethanol significantly eases shipping and handling concerns.

### EXAMPLE 5

It was discovered that water could be used to facilitate liquid-liquid separation of DET and ethylene glycol. 200g mixed PET flake (as described above), 600g ethanol (having 0.0734 wt% water) and 0.133g zinc acetate were charged to a 2-liter Parr reactor, heated to 220°C, stirred for 2 hours and cooled. Ethanol was removed from the reaction product mixture by distillation at atmospheric pressure followed by vacuum distillation of the remaining volatiles. The entire overhead from the vacuum distillation was collected as one fraction and weighed 207 grams. This fraction formed 2 liquid layers in a 58°C oven. The liquid layers were analyzed and the results set forth as 19a (lower layer) and 19b (upper layer) in Table 5 below. Water (41g) was then added and the mixture was shaken and allowed to settle into 2 layers. The layers were analyzed and the results set forth as 20a (lower layer) and 20b (upper layer) in Table 5 below.

| TABLE 5 Liquid-Liquid Separation | | | | |
|---|---|---|---|---|
| Run # | DET | Ethylene Glycol | Diethylene Glycol | Water |
| 19a | 95.69 wt% | 3.94 wt% | 0.27 wt% | 0 wt% |
| 19b | 4.29 wt% | 87.3 wt% | 4.79 wt% | 0 wt% |
| 20a | 96.03 wt% | 0.77 wt% | 0.28 wt% | 2.92 wt% |
| 20b | 0.45 wt% | 51.9 wt% | 3.09 wt% | 50.74 wt% |

As shown in Table 5, water enhances liquid-liquid separation between DET and ethylene glycol. The amount of ethylene glycol was significantly reduced in the lower layer and the amount of DET in the lower layer showed some increase. Significantly, most of the water remained in the upper layer with the bulk of the ethylene glycol. Because the water is primarily in the upper layer, it can be sent with ethylene glycol for further purification and the lower layer can be returned to distillation or isolated as finished DET product.

### EXAMPLE 6

It was discovered that paraxylene could be used to facilitate removal of DET from the ethylene glycol rich fraction by liquid-liquid extraction. 800 grams mixed PET flake (as described above), 2400 g ethanol (having 0.0734 wt% water) and 80 mg titanium (IV) isopropoxide were charged, in several batches, to a Parr reactor, heated to 200°C, stirred for 2 hours and cooled, For each batch, ethanol was removed from the reaction product mixture by distillation at atmospheric pressure followed by vacuum distillation of the remaining volatiles. The entire overhead from the vacuum distillation of the several batches was collected and combined as one fraction and weighed 947 grams. This fraction was treated in 4 steps. In step 1, this fraction formed 2 liquid layers in a 70°C oven. The lower layer was rich in DET and the upper layer was rich in ethylene glycol. The upper layer, which weighed 208 grams, was isolated. In step 2, 50 grams of water was added to the upper layer isolated in step 1. Addition of this water resulted in formation of two layers with the upper layer rich in ethylene glycol and weighing 214.6 grams and the lower layer rich in DET and weighing 43.4 grams. The step 2 upper layer was isolated and its composition is set forth in Table 6 below (Extraction 0). In step 3, a portion of the step 2 upper layer weighing 139 grams was mixed with an equal weight of paraxylene and the mixture was shaken and allowed to settle into 2 layers at 70°C. The lower layer was found to be rich in ethylene glycol and was isolated. The composition of this isolated step 3 lower layer is set forth in Table 6 below (Extraction 1). In step 4, the step 3 lower layer was mixed with an equal weight of fresh paraxylene, allowed to settle into 2 layers and the lower layer (rich in ethylene glycol) was isolated. The composition of the lower layer isolated in step 4 is reported below in Table 6 (Extraction 2).

| Table 6 Extraction of Ethylene Glycol with Paraxylene | | | | | |
|---|---|---|---|---|---|
| Extraction | DET (wt%) | Ethylene Glycol (wt%) | Diethylene Glycol (wt%) | water (wt%) | Paraxylene (wt%) |
| 0 | 0.50 | 79.18 | 1.93 | 18.4 | 0 |
| 1 | 0.0037 | 77.49 | 1.85 | 20.5 | 0.118 |
| 2 | <0.001 | 77.62 | 1.79 | 20.5 | 0.114 |

As shown in Table 6, extraction with paraxylene effectively removes DET from the glycol rich layer. The composition of the glycol rich layer, after extraction with a hydrocarbon such as paraxylene, is expected to be of sufficient purity as to allow further purification to polyester grade ethylene glycol by ordinary methods such as distillation.

### EXAMPLE 7

Batch liquid-phase oxidation reactions were performed using a 71 ml titanium batch reactor attached to a shaking device for agitation of the reactor contents. This reactor was charged with feedstock components as indicated in Table 6 below and a catalyst solution having 0.1 wt% Co + Mn (in the form of the acetates) and HBr in the reactor, at a molar ratio of Co/Mn/Br of 1/1/1. The solvent charged for comparative Run A and Runs 21 and 22 was a mixture of 80 wt% benzoic acid and water (20 wt%). The solvent charged for Run 23 was a mixture of 80 wt% acetic acid and 20 wt% water. The reactor was pressurized with air to yield 4.3 mols of O₂/mol of paraxylene charged. The reactor was then brought to the indicated temperature with agitation to provide the internal mixing. The reactor was held at the temperature for the indicated number of minutes, cooled to 25°C and both gas and slurry products were withdrawn and analyzed. High pressure liquid chromatography (HPLC) was used to analyze the total product. The acetic acid formation was determined by gas chromatography. The acetic acid yield was adjusted for the acetate present in the comparative Run A (introduced with the catalyst metals) with no correction for any acetic acid loss in the form of carbon oxides. The results (including comparative Run A) appear below in Table 7.

| TABLE 7 BATCH LIQUID-PHASE OXIDATION REACTIONS | | | | |
|---|---|---|---|---|
| | | | | |

| | A | 21 | 22 | 23 |
|---|---|---|---|---|
| **REACTOR CHARGE** | | | | |
| DET | 0.0000 | 0.1200 | 0.1200 | 0.1200 |
| Paraxylene | 0.5100 | 0.5000 | 0.5100 | 0.5100 |
| Water | 1.51 | 1.53 | 1.50 | 1.51 |
| Acetic Acid | | | | 7.50 |
| Benzoic Acid | 7.52 | 7.51 | 7.51 | 0 |
| | | | | |
| Temperature (°F) | 383 | 383 | 390 | 390 |
| Minutes @ Temp | 20 | 20 | 30 | 30 |
| Mol O₂/Mol Aromatic Hydrocarbon | 4.261 | 4.331 | 4.246 | 4.261 |
| | | | | |

| **PRODUCT** (wt%) | | | | |
|---|---|---|---|---|
| TA 1.00 | 8.05 | 7.1 | 7.85 | 6.75 |
| 4-CBA 1.122 | 0.049 | 0.047 | 0.023 | 0.018 |
| Benzoic acid 1.244 | 87.2 | 71.5 | 78.1 | <0.001 |
| p-Toluic acid 1.428 | D.026 | 0.031 | 0.005 | 0.004 |
| DET | 0 | 0.771 | 0.617 | 0.704 |
| MET | 0 | 0.298 | 0.357 | 0.275 |
| Mol % Ethyl Groups Converted | N/A | 33.9 | 53.5 | 70.2 |
| **Mols HOAc Gain/Mol Ethyl Groups Converted** | N/A | 52.2 | 59.6 | (not measured) |

The runs made with benzoic acid solvent allow measurement of the net formation of acetic acid which can be detected at low levels in the presence of benzoic acid solvent. These results indicate that in Run 21, 33.9% of the ethyl groups introduced with the DET were converted during the reaction period. This can be determined by the level of residual DET and MET in the product. The acetic acid in the product indicated that 52.2% of the ethyl groups converted appeared as net formation of acetic acid. In Run 22, using a slightly higher temperature (390F vs 383F) and longer reaction time (30 minutes vs 20 minutes), the ethyl group conversion increased to 53.5% and the selectivity to acetic acid formation increased to 59.6%.

in Run 23, because acetic acid was used as the solvent, it was not possible to accurately quantify the increase in acetic acid in the reactor. However, the conversion of 70% of the ethyl groups from DET and MET indicate a favorable conversion using this solvent.

As can be seen from the results in Table 7, use of DET as a feedstock component did not adversely affect the terephthalic acid production and, in Runs 22 and 23, resulted in significantly lower 4-CBA production. Example 6 illustrates that DET can be used as a substitute for all or part of paraxylene typically used as feedstock for the liquid phase production of terephthalic acid.

### EXAMPLE 8

Semi-continuous liquid-phase oxidation was conducted using a 2 liter stirred pressure reactor constructed of titanium. This unit was charged with solvent and catalyst only, pressurized under nitrogen, and heated to the indicated reaction temperature with stirring at 1000 RPM. A feedstock mixture of 20 wt% DET and 80wt% paraxylene was then added to the reactor at a rate of 333 grams over 80 minutes. During this period, a gas stream comprised of 21 wt% O₂ in nitrogen was also directed into the bottom of the reactor and gas leaving the reactor was passed through a condenser to return condensable solvent to the reactor while venting non-condensable gaseous components. After all of the DET/paraxylene feedstock had

been added, the gas was changed back to nitrogen, the unit cooled, and the product collected and analyzed as in previous example. The results appear in Table 8 below.

| TABLE 8 SEMI-CONTINUOUS LIQUID PHASE | | |
|---|---|---|
| | | |
| | 24 | 25 |

| Reactor Charge (Solvent contained 880 ppm Co and Co/Mn/Br at 1/1/1 molar ratio) | | |
|---|---|---|
| DET | 67 | 67 |
| Paraxylene | 266 | 266 |
| Acetic Acid | 0 | 884 |
| Benzoic Acid | 884 | 0 |
| Water | 130 | 130 |
| Reactor Temperature(F) | 385 | 385 |
| % Conversion of (DET+MET) | 60.2 | 51.7 |
| | | |

| Product (wt%) | | |
|---|---|---|
| TA | 37.0 | 31.0 |
| 4-CBA | 0.03 | 0.080 |
| BENZOIC ACID (BA) | 58.8 | 0.135 |
| p- TOLUIC ACID | <0.001 | 0.051 |
| p-TOLUALDEHYDE | <0.001 | <0.001 |
| | | |
| DET | 0.788 | 0.993 |
| MET | 1.25 | 1.33 |

Table 8 illustrates that DET can be used successfully as a portion of the feedstock in a process for the liquid phase oxidation of paraxylene to produce terephthalic acid with low 4-CBA values and with greater than 50% conversion of the DET/MET mixture per pass.
The present inventions are also defined by way of the following clauses:
1. A process for recycling poly(ethylene terephthalate) comprising the steps of:
   a) reacting in a reaction zone poly(ethylene terephthalate) with ethanol to form a reaction product mixture;
   b) recovering from the reaction product mixture a first fraction comprising recovered ethanol;
   c) recovering from the reaction product mixture a second fraction comprising ethylene glycol; and d) recovering from the reaction product mixture a third fraction comprising diethyl terephthalate.
2. The process of clause 1 wherein the ethanol in the step of combining in a reaction zone poly(ethylene terephthalate) with ethanol to form a reaction mixture comprises fuel grade ethanol.
3. The process of clause 1 wherein the step of recovering from the reaction product mixture a first fraction comprising recovered ethanol is performed in a first separation zone and the steps of recovering from the reaction product mixture a second fraction comprising ethylene glycol and recovering from the reaction product mixture a third fraction comprising diethyl terephthalate are performed in a second separation zone.
4. The process of clause 3 further comprising the steps of:
   e) separating the second fraction into a first stream comprising a major portion of diethyl terephthalate and a second stream comprising ethylene glycol;
   f) returning at least a portion of the first stream to the second separation zone; and
   g) recovering ethylene glycol from the second stream in a third separation zone.
5. The process of clause 4 wherein the step of separating the second fraction comprises the step of adding water to at least a portion of the second fraction.
6. The process of clause 5 wherein the step of separating the second fraction comprises the step of adding n-heptane, paraxylene or both to at least a portion of the second fraction.
7. The process of clause 3 wherein the first separation zone comprises a first distillation column operated at about atmospheric pressure and the second separation zone comprises a second distillation column operated at a pressure less than atmospheric pressure.
8. The process of clause 3 wherein at least a portion of the recovered ethanol in the first fraction is present in the reaction zone.
9. The process of clause 1 wherein catalyst is present in the reaction zone and the catalyst is selected from the group consisting of catalyzing impurities, copper phthalocyanine, zinc, cobalt, manganese, magnesium, titanium, and combinations thereof.
10. The process of clause 9 wherein catalyst is present in the reaction zone and the catalyst comprises titanium.
11. The process of clause 10 wherein the ethanol in the step of combining in a reaction zone poly(ethylene terephthalate) with ethanol to form a reaction mixture comprises fuel grade ethanol.
12. The process of clause 9 further comprising the step of recovering from the reaction product mixture a fourth fraction comprising catalyst wherein at least a portion of the fourth fraction is directed to the reaction zone.
13. The process of clause 1 wherein at least a portion of the reaction product mixture is subjected to solid-liquid separation to remove at least a portion of undesired contaminants.
14. The process of clause 1 wherein at least a portion of the reaction product mixture is subjected to ion exchange to remove at least a portion of undesired contaminants.
15. An apparatus for the recycle of poly(ethylene terephthalate) comprising:
   a) a reactor capable of reacting poly(ethylene terephthalate) and ethanol and forming a reaction product mixture;
   b) an atmospheric distillation column adapted to recover ethanol from the reaction product mixture and return at least a portion of the recovered ethanol directly or indirectly to the reactor; and
   c) a vacuum distillation column adapted to recover diethyl terephthalate from the reaction product mixture.
16. The apparatus of clause 15 further comprising a solid-liquid separation device capable of removing at least a portion of insoluble undesired contaminants from at least a portion of the reaction product mixture.
17. The apparatus of clause 15 further comprising an ion exchange resin capable of removing at least a portion of soluble undesired contaminants from at least a portion of the reaction product mixture.
18. A process for the production of diethyl terephthalate comprising the steps of:
   a) reacting poly(ethylene terephthalate) and ethanol in a reaction zone to form a reaction product mixture comprising ethanol, poly(ethylene terephthalate), diethyl terephthalate and ethylene glycol;
   b) separating from the reaction product mixture a first fraction comprising ethanol, a second fraction comprising a diethyl terephthalate - ethylene glycol azeotrope and a third fraction comprising diethyl terephthalate.
19. The process of clause 18 wherein water is present in the reaction zone.
20. The process of clause 18 further comprising the steps of:
   c) recovering from the azeotrope a stream comprising a major portion of diethyl terephthalate using liquid-liquid separation at a temperature above the melting point of diethyl terephthalate; and
   d) directing at least a portion of the stream of step (c) to separation in step (b).
21. The process of clause 20 further comprising step of separating at least a portion of insoluble undesired contaminants from the reaction product mixture.
22. The process of clause 20 further comprising the step of separating, using ion exchange, at least a portion of soluble undesired contaminants from the reaction product mixture.
23. The process of clause 18 wherein catalyst is present in the reaction zone and the catalyst is selected from the group consisting of catalyzing impurities, copper phthalocyanine, zinc, cobalt, manganese, magnesium, titanium and combinations thereof.
24. The process of clause 23 wherein the catalyst comprises titanium(IV) isopropoxide.
25. A process for producing diethyl terephthalate and diethyl isophthalate comprising the steps of:
   a) reacting in a reaction zone ethanol with a feed comprising a terpolymer of terephthalic acid, isophthalic acid, and ethylene glycol to form a reaction product mixture;
   b) recovering from the reaction product mixture a first fraction comprising ethanol;
   c) recovering from the reaction product mixture a second fraction comprising ethylene glycol; and
   d) recovering from the reaction product mixture a third fraction comprising diethyl terephthalate and diethyl isophthalate.
26. The process of clause 25 wherein catalyst is present in the reaction zone and the catalyst is selected from the group consisting of catalyzing impurities, copper phthalocyanine, zinc, cobalt, manganese, magnesium, titanium and combinations thereof.
27. The process of clause 25 wherein the ethanol in the reaction zone comprises fuel grade ethanol.
28. A feedstock for the production of aromatic carboxylic acid comprising at least one aromatic ethyl ester.
29. The feedstock of clause 28 wherein the at least one aromatic ethyl ester comprises an aromatic diethyl ester.
30. The feedstock of clause 29 wherein the aromatic diethyl ester is diethyl terephthalate.
31. The feedstock of clause 30 further comprising diethyl isophthalate.
32. The feedstock of clause 28 wherein the at least one aromatic ethyl ether comprises diethyl naphthalene.
33. The feedstock of clause 28 further comprising at least one dimethyl aromatic hydrocarbon.
34. The feedstock of clause 33 wherein the at least one aromatic ethyl ester comprises diethyl terephthalate and the at least on dimethyl aromatic hydrocarbon comprises paraxylene.
35. The feedstock of clause 34 further comprising diethyl isophthalate.
36. A method of producing aromatic carboxylic acids comprising the step of reacting in a reaction zone at least one aromatic ethyl ester and oxygen in the presence of a solvent comprising acetic acid.
37. The method of clause 36 wherein the aromatic ethyl ester comprises diethyl terephthalate.
38. The method of clause 37 wherein paraxylene is present in the reaction zone.
39. The method of clause 38 further comprising the steps of:
   a) withdrawing from the reaction zone a reaction product mixture comprising diethyl terephthalate and terephthalic acid;
   b) separating the reaction product mixture to recover terephthalic acid product and form reaction mother liquor comprising diethyl terephthalate; and
   c) returning at least a portion of the reaction mother liquor to the reaction zone.
40. The method of clause 39 wherein the oxidation mother liquor comprises mono- ethyl terephthalate.
41. A method for producing acetic acid comprising the step of reacting in a reaction zone at least one aromatic ethyl ester and oxygen in the presence of water.
42. The method of clause 41 wherein the at least one aromatic ethyl ester comprises diethyl terephthalate.
43. The method of clause 42 wherein the at least one aromatic ethyl ester further comprises diethyl isophthalate.
44. A method of co-producing aromatic carboxylic acid and acetic acid, the method comprising reacting in a reaction zone a feedstock comprising at least one aromatic ethyl ester with oxygen.
45. The method of clause 44 wherein the aromatic carboxylic acid comprises terephthalic acid and the at least one aromatic ethyl ester comprises diethyl terephthalate.
46. The method of clause 45 wherein paraxylene is present in the reaction zone.
47. A process for recycling poly(ethylene terephthalate) comprising the steps of:
   a) reacting, in a first reaction zone, a first feed comprising poly(ethylene terephthalate) with ethanol to form a first reaction product mixture;
   b) recovering aromatic ethyl esters from the first reaction product mixture;
   c) oxidizing, in a second reaction zone, a second feed comprising at least a portion of the aromatic ethyl esters to form aromatic carboxylic acid; and
   d) reacting, in a third reaction zone, at least a portion of the aromatic carboxylic acid and ethylene glycol to form a polymer comprising poly(ethylene terephthalate).
48. The process of clause 47 wherein the first feed comprises at least 1000 ppmw polyvinylchloride (on a poly(ethylene terephthalate) basis).
49. The process of clause 48 wherein at least a portion of the first reaction product mixture is contacted with an ion exchange resin to remove at least a portion of soluble contaminants present in the first reaction product mixture.
50. The process of clause 47 wherein the ethanol is fuel grade ethanol.
51. The process of clause 47 wherein the second feed comprises a dimethyl aromatic hydrocarbon precursor of the aromatic carboxylic acid.

## Claims

1. A method of producing aromatic carboxylic acids comprising the step of reacting in a reaction zone at least one aromatic ethyl ester and oxygen in the presence of a solvent comprising acetic acid.

2. The method of Claim 1 wherein the aromatic ethyl ester comprises diethyl terephthalate.

3. The method of Claim 2 wherein paraxylene is present in the reaction zone.

4. The method of Claim 3 further comprising the steps of:
a) withdrawing from the reaction zone a reaction product mixture comprising diethyl terephthalate and terephthalic acid;
b) separating the reaction product mixture to recover terephthalic acid product and form reaction mother liquor comprising diethyl terephthalate; and
c) returning at least a portion of the reaction mother liquor to the reaction zone.

5. The method of Claim 4 wherein the oxidation mother liquor comprises mono-ethyl terephthalate.

6. A method for producing acetic acid comprising the step of reacting in a reaction zone at least one aromatic ethyl ester and oxygen in the presence of water.

7. The method of Claim 6 wherein the at least one aromatic ethyl ester comprises diethyl terephthalate.

8. The method of Claim 7 wherein the at least one aromatic ethyl ester further comprises diethyl isophthalate.

9. A method of co-producing aromatic carboxylic acid and acetic acid, the method comprising reacting in a reaction zone a feedstock comprising at least one aromatic ethyl ester with oxygen.

10. The method of Claim 9 wherein the aromatic carboxylic acid comprises terephthalic acid and the at least one aromatic ethyl ester comprises diethyl terephthalate.

11. The method of Claim 10 wherein paraxylene is present in the reaction zone.

12. A feedstock for the production of aromatic carboxylic acid comprising at least one aromatic ethyl ester and further comprising at least one dimethyl aromatic hydrocarbon.

13. The feedstock of Claim 12 wherein the at least one aromatic ethyl ester comprises an aromatic diethyl ester.

14. The feedstock of Claim 13 wherein the aromatic diethyl ester is diethyl terephthalate.

15. The feedstock of Claim 14 further comprising diethyl isophthalate.

16. The feedstock of Claim 12 wherein the at least one aromatic ethyl ether comprises diethyl naphthalene.

17. The feedstock of Claim 12 wherein the at least one aromatic ethyl ester comprises diethyl terephthalate and the at least on dimethyl aromatic hydrocarbon comprises paraxylene.

18. The feedstock of Claim 17 further comprising diethyl isophthalate.

19. A method according to any one of Claims 2 to 5, 7 to 8 and 10 to 11 wherein the diethyl terephthalate is prepared by a process comprising the steps of:
i) reacting in a reaction zone poly(ethylene terephthalate) with ethanol to form a reaction product mixture;
ii) recovering from the reaction product mixture a first fraction comprising recovered ethanol;
iii) recovering from the reaction product mixture a second fraction comprising ethylene glycol; and
iv) recovering from the reaction product mixture a third fraction comprising diethyl terephthalate.

20. An apparatus for the method of Claim 19 comprising:
a) a reactor capable of reacting poly(ethylene terephthalate) and ethanol and forming a reaction product mixture;
b) an atmospheric distillation column adapted to recover ethanol from the reaction product mixture and return at least a portion of the recovered ethanol directly or indirectly to the reactor; and
c) a vacuum distillation column adapted to recover diethyl terephthalate from the reaction product mixture.

21. A method according to any one of Claims 2 to 5, 7 to 8 and 10 to 11 wherein the diethyl terephthalate is produced by a process comprising the steps of:
i) reacting poly(ethylene terephthalate) and ethanol in a reaction zone to form a reaction product mixture comprising ethanol, poly(ethylene terephthalate), diethyl terephthalate and ethylene glycol;
ii) separating from the reaction product mixture a first fraction comprising ethanol, a second fraction comprising a diethyl terephthalate - ethylene glycol azeotrope and a third fraction comprising diethyl terephthalate.

22. A method according to any one of Claims 2 to 5, 7 to 8 and 10 to 11 wherein the diethyl terephthalate is produced by a process comprising the steps of:
i) reacting in a reaction zone ethanol with a feed comprising a terpolymer of terephthalic acid, isophthalic acid, and ethylene glycol to form a reaction product mixture;
ii) recovering from the reaction product mixture a first fraction comprising ethanol;
iii) recovering from the reaction product mixture a second fraction comprising ethylene glycol; and
iv) recovering from the reaction product mixture a third fraction comprising diethyl terephthalate and diethyl isophthalate.

23. A process for recycling poly(ethylene terephthalate) comprising the steps of:
i) reacting, in a first reaction zone, a first feed comprising poly(ethylene terephthalate) with ethanol to form a first reaction product mixture;
ii) recovering aromatic ethyl esters from the first reaction product mixture;
iii) oxidizing, in a second reaction zone, a second feed comprising at least a portion of the aromatic ethyl esters to form aromatic carboxylic acid; and
iv) reacting, in a third reaction zone, at least a portion of the aromatic carboxylic acid and ethylene glycol to form a polymer comprising poly(ethylene terephthalate).
